# EUROPEAN PATENT APPLICATION

(11) **EP 4 306 125 A1**
(43) Date of publication of application: **17.01.2024**
(21) Application number: 22209762.8
(22) Date of filing: 26.11.2022
(51) Int. Cl.: A61K 39/00, A61P 35/00, G01N 33/00

(54) **IMMUNOGENIC PERSONALISED CANCER VACCINES**

(30) Priority: 14.07.2022 EP 22185038
(71) Applicant: Universität Zürich, 8006 Zürich (CH)
(72) Inventor: MARTIN, Roland, 8006 Zürich (CH)
(74) Representative: Schulz Junghans Patentanwälte PartGmbB

(57) **Abstract**

The invention relates to a method of obtaining a variant of a T cell epitope peptide expressed in the tumour of cancer patient, wherein the variant is characterised by targeted amino acid substitutions conferring an improved capacity to stimulate CD8+ and CD4+ T cells. The invention further relates to vaccine and, pharmaceutical compositions comprising variant T cell epitope peptides and their use in treating cancer.

## Description

The present invention relates to methods of generating artificial immunogenic peptides capable of stimulating a T cell response to self-antigens expressed in patient tumours, and the use of such immunogenic peptides in treating cancer.

### Background of the Invention

Personalized vaccination approaches for treating cancer should be optimized with respect to the tumour characteristics of each patient, including mutations, gene and protein expression profiles, and the individual human leukocyte antigen (HLA) type driving CD4+ and CD8+ T cell responses.

CD8⁺ T cells are best known for their cytotoxic function, critical for anti-tumour immune responses. However, it is becoming increasingly recognized that effective CD4⁺ T cell responses are also indispensable for tumour control or eradication. Based on data from tumour-specific immune responses in animal models as well as human tumours, a personalized vaccination approach should stimulate both tumour-specific CD4⁺ and CD8⁺ T cells (Dobrzanski M., Front. Oncol 2013, 3:63; Melief C. J., Nat. Rev. Cancer 2008, 8:351). CD4⁺ T cells are important for activating innate immune cells, generating a proinflammatory environment as well as the recruitment of other immune cells, and providing help to CD8⁺ effector T cells. In the context of tumours they can also generate an efficient cytotoxic response as shown for eliminating virus-infected cells or killing target cells expressing autoantigens. Recent promising data with a mutation-specific peptide vaccine for IDH1-mutant glioma underscore the importance of tumour antigen-specific CD4⁺ T cells (Platten M. Nature 2021, 592:463; Schumacher T. Nature 2014, 512:324).

A key aspect for inducing efficient immune responses is identifying putative tumour antigens that can be employed in the respective vaccination approach. Mutations and proteins that are highly expressed in the tumour are the most desirable targets. However, these proteins evoke only weak and low avidity T cell responses due to negative selection of T cells with high avidity self-reactivity in the thymus (Klein L. Nat,. Med. 2000, 6:56). In contrast, mutated tumour proteins appear "foreign" to T cells, eliciting strong immune responses because T cells with specificity for these mutated proteins have not been deleted by negative selection. Accordingly, so-called "hot" tumours with high mutational load like malignant melanoma generally elicit strong anti-tumour T cell responses, while glioblastoma, an immunologically "cold" tumour with fewer somatic mutations, is usually less immunogenic, proving resistant to existing peptide vaccination strategies (Schumacher T. N., Science 2015, 348:69; Klemm F. Cell 2020, 181:1643; Agliardi G., Nat. Comm. 2021, 12:444; Yarchoan M., N. Engl. J. Med. 2017, 377:2500).

Based on the above-mentioned state of the art, the objective of the present invention is to provide means and methods to obtain improved peptides for use in cancer vaccines that effectively break immune tolerance to self-antigens expressed within tumours. This objective is attained by the subject-matter of the independent claims of the present specification, with further advantageous embodiments described in the dependent claims, examples, figures and general description of this specification.

### Summary of the Invention

To improve vaccination outcomes for tumours with low mutational load and high levels of T cell tolerance, a highly personalized vaccination strategy was developed to obtain neopeptides, that is designer peptides bearing targeted amino acid substitutions. The immune response of peripheral blood T cells to vaccine peptides, including natural peptides and designed neopeptides, is shown to be gradually increased in a patient receiving repetitive administration of such neopeptides. Tumour-infiltrating lymphocytes (TIL) responded strongly to all vaccine peptides designed for HLA class II molecules and 45% designed for HLA class I molecules. TIL-derived CD4⁺ T cell clones showed that designed neopeptides elicited both strong CD4⁺ T cell responses against the related unmutated natural peptides on which the sequence is based, and could cause tumour cell lysis. Thus, turning tumour self-peptides into foreign antigens is a promising strategy to induce intratumoural T cell responses in immune cold tumours, such as glioblastoma.

A first aspect of the invention relates to a method of converting a candidate tumour T cell epitope peptide sequence into a neopeptide sequence recognised as foreign by the patient's immune system due to the presence of at least one targeted amino acid substitution. The method comprises as a first step selecting a candidate T cell antigen peptide sequence either likely to be expressed in certain form of cancer, such as glioblastoma, or demonstrated to be expressed at the gene or protein level in a tumour sample obtained from a specific cancer patient for whom a personalised vaccine is being designed. The candidate T cell antigen peptide sequence comprises an HLA-binding motif of 8 to 11, particularly 9 or 10 amino acids, predicted to bind to a human leukocyte antigen (HLA) allele expressed as a protein by a cancer patient in need of a personalised vaccine component. Next, HLA-anchor positions required for binding to an HLA molecule present in the patient are identified within the candidate peptide sequence, which must be preserved to retain presentation of a variant neopeptide to T cell receptors. Finally, amino acid substitutions are introduced within the candidate T cell antigen peptide sequence, placed so as to avoid HLA-anchor positions predicted to mediate binding of the candidate T cell antigen peptide to one of the patient's HLA allele molecules, to provide an improved neopeptide sequence. At least one mutation is located within the amino acids of the neopeptide sequence corresponding to the HLA-binding motif of the candidate peptide sequence. One or more of such neopeptide sequences can be synthesised and/or incorporated into a vaccine preparation, demonstrated to effectively stimulate anti-tumour CD4+ and CD8+ T cell responses in the examples provided.

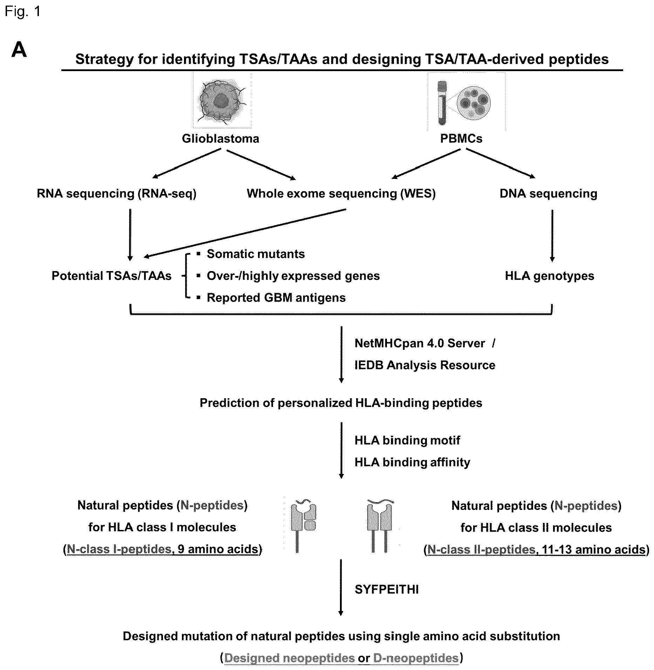

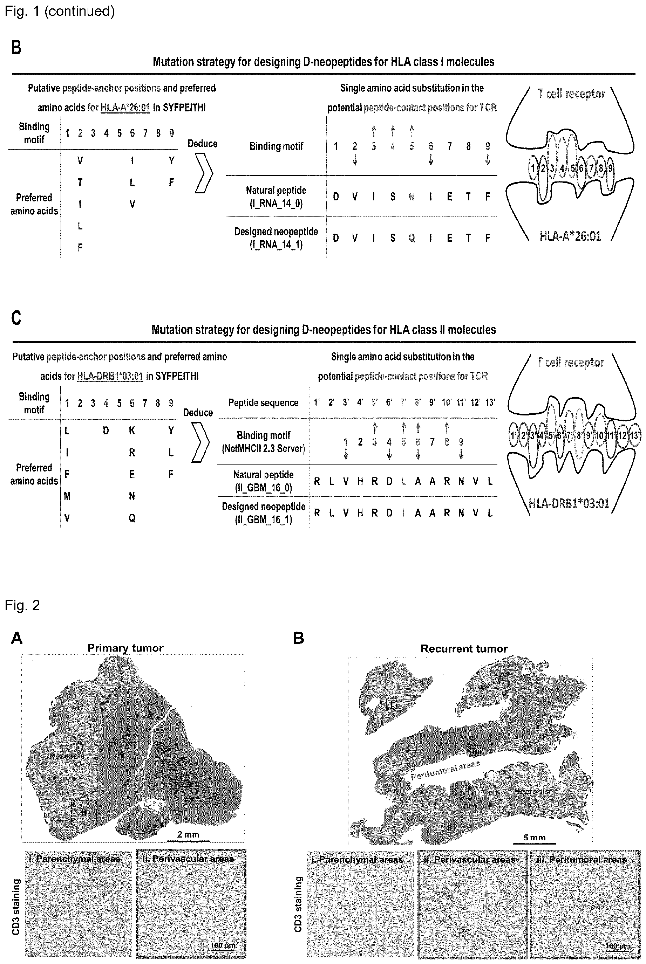

Another aspect of the invention relates to a neopeptide comprising an HLA-binding motif of 8 to 11, particularly 9 or 10 amino acids long, with the capacity to bind to an HLA allele molecule present in a cancer patient and activate CD4+ or CD8+ T cells. The amino acid sequence of the neopeptide is a variant of a candidate peptide sequence expressed by a cancer patient's tumour at the gene or protein level, or expressed commonly in a certain cancer tissue. The neopeptide is characterised by a peptide sequence comprising one or two amino acid substitutions in comparison to the candidate peptide sequence, at least one of which is located at a central position within the HLA-binding motif comprised within the peptide, and avoiding HLA-anchor amino acids essential for restriction, i.e. processing and presentation by, an HLA protein present in the patient (Fig. 1)

In particular embodiments of the method or neopeptide as specified in the preceding two paragraphs, the neopeptide sequence is characterised by an amino acid substitution located at one of the central amino acids within the HLA-binding motif, particularly adjacent to conserved HLA-anchor amino acids. In some embodiments, the neopeptide sequence is a CD4+ T cell antigen peptide 10-25 amino acids in length, comprising an HLA-binding motif binding an HLA class II allele protein present in the cancer patient. In other embodiments, the neopeptide sequence is a CD8+ T cell antigenic peptide 9-11 amino acids in length, comprising at least one HLA-binding motif binding an HLA class I allele protein present in the cancer patient. The amino acid substitution may be conservative, neutral or non-conservative compared to the equivalent residue in the candidate peptide sequence on which it is based.

In particular embodiments, the candidate peptide sequence on which the variant neopeptide sequence is based is derived from a gene, or protein characterised by upregulation, or a high expression level, in a tumour sample obtained from the patient, and/or contains a tumour-specific somatic mutation. In other embodiments, the candidate peptide sequence is present in a protein with above average expression in cohort of cancer patients diagnosed with the same class of cancer as the cancer patient for which the personalised neopeptide sequence is being developed. In particular embodiments, the patient's tumour is a type of cancer associated with low mutation loads, or an immune cold cancer, such as glioblastoma.

Another aspect of the invention is a vaccine composition comprising, in peptide form, or formulated for delivery as a nucleic acid, a neopeptide with a neopeptide sequence obtained by the method as specified in the first aspect of the invention, or comprising a neopeptide as specified in the second aspect of the invention, in particular comprising a plurality of such neopeptides designed to activate both CD4+ and CD8+ T cells. In another embodiment, the present invention relates to a pharmaceutical composition comprising at least one of the neopeptides of the present invention or a pharmaceutically acceptable salt thereof, and at least one pharmaceutically acceptable carrier, diluent or excipient. The invention further encompasses use of such neopeptides, vaccine compositions or pharmaceutical compositions for treating cancer, and methods of treating cancer patients by administering an effectively immunogenic amount of a cancer vaccine comprising neopeptides according to the invention.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

### Sequences

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Centre for Biotechnology-Information (http://blast.ncbi.nlm.nih.gov/). Reference to identical sequences without specification of a percentage value implies 100% identical sequences (i.e. the same sequence).

A useful method for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* in the context of the present specification relates to a molecule consisting of in particular 8 to 30 amino acids that form a linear chain wherein the amino acids are connected by peptide bonds. Peptides of use according to the invention are T cell epitopes, which may bind to, and be presented by, HLA molecules expressed on the surface of cell, particularly a tumour cell or an antigen presenting cell. An internal binding motif called an HLA-binding motif, (also termed a binding register) region of approximately 8-11 amino acids within the peptide interacts with T cell receptors in the context of HLA presentation. Peptides binding to HLA allele polypeptides of particular use according to the invention to activate CD4+ and CD8+ T cells are generally 8 to 25 amino acids in length.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). Citrulline (Cit) is (2S)-2-amino-5-(carbamoylamino)pentanoic acid (CAS No. 372-75-8).

The term *variant* refers to a peptide or polypeptide that differs from a reference peptide or polypeptide, but retains essential properties. A typical variant of a polypeptide differs in its primary amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may or may not be one encoded by the genetic code. A variant of a polypeptide may be naturally occurring such as an HLA allelic variant, or a somatic mutated protein, or it may be a variant that is not known to occur naturally, such as a neopeptide. Neopeptide variants according to the invention, can be of variable length compared to the sequence on which they are based, however, in order to retain HLA-binding capacity the core HLA-binding motif should be the same length, and can thus only be characterised by amino acid substitutions, rather than amino acid additions and deletions.

### General Molecular Biology: Nucleic Acid Sequences, Expression

The term gene refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

### Cell Biology, diagnostic method inventions: Markers, ligands

In the present specification, the term *positive,* when used in the context of expression of a marker, refers to expression of an antigen assayed by a fluorescently labelled antibody, wherein the label's fluorescence on the structure (for example, a cell) referred to as "positive" is at least 30% higher (≥ 30 %), particularly ≥50% or ≥80%, in median fluorescence intensity in comparison to staining with an isotype-matched fluorescently labelled antibody which does not specifically bind to the same target. Such expression of a marker is indicated by a superscript "plus" (⁺), following the name of the marker, e.g. CD4⁺, or by the addition of the plus sign after the marker name ("CD4+"). If the word "expression" is used herein in the context of "gene expression" or "expression of a marker or biomolecule" and no further qualification of "expression" is mentioned, this implies "positive expression" as defined above.

In the present specification, the term *negative,* when used in the context of expression of a marker, refers to expression of an antigen assayed by a fluorescently labelled antibody, wherein the median fluorescence intensity is less than 30% higher, particularly less than 15% higher, than the median fluorescence intensity of an isotype-matched antibody which does not specifically bind the same target. Such expression of a marker is indicated by a superscript minus (⁻), following the name of the marker, e.g. CD127⁻, or by the addition of the minus sign after the marker name ("CD3-").

*High* expression of a marker, for example high expression of, for example, an activation marker such as CD25, refers to the expression level of such marker in a clearly distinguishable cell population that is detected by FACS showing the highest fluorescence intensity per cell compared to the other populations characterized by a lower fluorescence intensity per cell. A high expression is indicated by superscript "high" or "hi" following the name of the marker, e.g. CD25^{high}. The term "is expressed highly" refers to the same feature.

*Low* expression of a marker, for example low expression of CD25, refers to the expression level of such marker in a clearly distinguishable cell population that is detected by FACS showing the lowest fluorescence intensity per cell compared to the other populations characterized by higher fluorescence intensity per cell. A low expression is indicated by superscript "low" or "lo" following the name of the marker, e.g. CD25^{low}. The term "is expressed lowly" refers to the same feature.

The expression of a marker may be assayed via techniques such as fluorescence microscopy, flow cytometry, ELISPOT, ELISA or multiplex analyses.

### Binding, HLA binding, HLA binding peptides

The term *HLA* in the context of the present specification relates to the products on the major histocompatibility complex (MHC), in humans known as the human leukocyte antigen (HLA) located on chromosome 6, having the biological role of presenting processed peptide antigens to specifically reactive T cell receptors on T cells. Polymorphic expression of *HLA* among humans requires molecular HLA typing of patients to be routinely performed in the clinic, for example in transplantation procedures, to identify the specific HLA class I and class II alleles expressed as proteins, inherited in a Mendelian fashion from each parent.

The term *HLA class I allele* in the context of the present specification relates to the gene, or gene product of the HLA class I gene family mediating antigen presentation to CD8+ T cells. *HLA class I allele* HLA-A, HLA-B and HLA-C genes encode glycosylated heavy chains which associate non covalently with an invariant beta-2-microglobulin chain to provide a membrane bound structure forming a peptide-binding groove between two alpha helices and a beta-pleated floor, to provide a binding site for processed peptide antigen from within the cell. The class I peptide binding groove accommodates peptides 8 to 10, usually 9 amino acids in length.

The term *HLA class II allele* in the context of the present specification relates to the genes, or the gene product of the HLA class I gene family, which mediate antigen presentation to CD4+ T cells. The *HLA class II allele DR, DP, and DQ* subfamilies encode glycosylated alpha and beta heavy chains which associate as heterodimers to form a membrane bound structure creating a peptide-binding groove between the alpha 1 domain and the beta 1 domain, to provide a binding site for exogenously derived peptide antigen. The class II peptide binding groove accommodates longer peptides 8 to 25, usually around 12 amino acids in length, and these longer peptides may comprise one or more 8-11 amino acid HLA-binding motifs with the capacity to bind to an HLA peptide binding groove.

The peptide-binding specificity of each HLA class I or class II allele is determined by a limited number of amino acids present within the peptide-binding groove, or pocket. The peptide-binding groove of an HLA molecule binds to a peptide at aspecific motif of 8 to 11 amino acids (usually 9 or 10) referred to herein as an *HLA-binding motif.* Each HLA molecule binds to characteristic amino acid residues patterns in any bound peptide sequences. These conserved residues at particular positions are essential for binding of the *HLA-binding motif* of the peptide to the peptide binding groove of an HLA molecule, and are referred to in the specification as *HLA-anchor amino acids.* Figure 1 of the examples illustrates an example of preferred amino acids for a representative HLA allele which are *HLA-anchor amino acids* according to the invention. Such HLA-anchor amino acids unique to each HLA class II or I allele may be identified by means of an *HLA-anchor prediction algorithm* (see below). In addition, in optional embodiments, the two N- terminal amino acids, and the two C-terminal amino acids of the HLA-binding motif are assigned according to the invention as HLA-anchor amino acids.

The term *HLA-anchor prediction algorithm* in the context of the present specification relates to software configured to align an input candidate peptide sequence with a database of conserved amino acid motifs present in all peptides binding a certain HLA allele. Any such conserved amino acid is assigned as an HLA anchor HLA-anchor acid according to the invention (see above). By this means, the algorithm can identify conserved amino acid motifs in the peptide sequence of a T cell epitope which are predicted to mediate binding, and/or interact very closely with the peptide binding groove of a specific HLA class I allele, or HLA class II allele molecule. Freely available online tools such as SYFPEITHI may provide this assignment.

The term *tumour antigen* in the context of the present specification relates to a protein expressed by cancer cells present within a tumour. It encompasses both *tumour antigens* commonly present in certain types of cancers, for example, testis antigens, or proteins which have been detected (either at the level of protein, or at gene expression) in a tumour sample obtained from a cancer patient. Tumour antigens may, or may not comprise a tumour-specific, non-inherited mutation.

The term *candidate peptide sequence* in the context of the present specification relates to an amino acid sequence present within a protein sequence expressed in a patient tumour sample, measured either at the level of mRNA expression, or protein expression, or identified by analysis of the immunopeptidome of a patient's tumour cells. Alternatively, a *candidate peptide sequence* is comprised within the protein sequence of a tumour associated antigen commonly expressed in certain forms of cancer, in other words expressed at a greater level in tumour samples obtained from a cohort of cancer patients diagnosed with the same form of cancer, compared to samples obtained from healthy tissue of a cohort of control subjects. *Candidate peptide sequence* are 8 to 25 amino acids in length, and comprise an HLA-binding motif conferring to the peptide encoded by the sequence a specific interaction with an HLA allele expressed in the cancer patient, facilitating presentation to CD4+ or CD8+ T cells and the induction of an immune response.

The term *neopeptide* in the context of the present specification relates to a peptide designed as a personalised medicament be presented on HLA molecules to activate CD4+ or CD8+ T cells reactive to tumour cells found in a cancer patient. Like the candidate peptide sequence on which they are based, neopeptides are 8 to 25 amino acids in length, and comprise one or more HLA-binding motifs conferring specific, or cognate interaction with an HLA allele present in the cancer patient, facilitating presentation to CD4+ or CD8+ T cells and the induction of an immune response. A neopeptide is characterised by a neopeptide amino acid sequence (*neopeptide sequence*), a variant peptide sequence characterised by one, or at most two amino acid substitutions in comparison to a candidate peptide sequence of the preceding paragraph. The placement and characteristics of these amino acid substitutions is the basis of the current invention and is addressed at length in the section entitled *Methods to obtain neopeptides.*

The term *specific binding* in the context of the present invention refers to a property of peptides within a candidate peptide sequence designated as the HLA-binding motif. This HLA-binding motif comprises 8 to 10 amino acid region that binds to an HLA molecule with a certain affinity and target specificity. The affinity of this interaction can be indicated by the *dissociation constant (KD)* of the peptide and the HLA molecule. A specifically reactive ligand has a dissociation constant of ≤ 10⁻⁷mol/L when binding to its target, but a dissociation constant at least three orders of magnitude higher in its interaction with a molecule having a globally similar chemical composition as the target, but a different three-dimensional structure (the same amino acids in the peptide, but ordered in a different sequence). In the context of the present specification, the term *dissociation constant (KD)* is used in its meaning known in the art of chemistry and physics; it refers to an equilibrium constant that measures the propensity of a complex composed of [mostly two] different components to dissociate reversibly into its constituent components. *Dissociation constant* of a peptide in relation to an HLA allele is frequently approximated by means of an IC50 value, defined as the concentration of peptide yielding a 50% inhibition of binding of a labelled peptide with known high affinity binding to the HLA complex. For the purposes of the methods specified herein, *specific binding* is to be determined by means of an *in silico* prediction of an IC50 value, by means of the freely available online T cell immune epitope prediction software provided by SYFPEITHI (Rammensee et al., 1999 Immunogen. 50(3-4):213). The service predicts the ligation strength to a defined HLA type for a sequence of amino acids. The predictive algorithms are derived from "MHC Ligands and Peptide Motifs" by H.G.Rammensee, J.Bachmann and S.Stevanovic, and assess consecutive overlapping peptides of a specified length within an protein sequence input, for example overlapping 9-mers, as to their likelihood of being a strong, weak, or non-binder to a specified HLA class I or class II allele molecule, and thus whether they may be a candidate T cell epitope of use as a candidate peptide sequence according to the invention. HLA-anchor amino acids are also indicated in the results. Strong binders are those more likely to display high binding to said HLA, for example an IC50 value < 50nM, whereas a non-binder is characterised by a higher IC50 value, for example, over 1000nM, or even over 1500nM. A commonly applied cut-off for specific binding is 500nM for a peptide binding to a HLA class I molecule, whereas the due to the generally weaker associations of peptides for HLA class II, a higher cut off of 1000nM is often applied.

### Pharmaceutical compositions and treatment

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavouring agents, dyes, and the like and combinations thereof, as would be known to those skilled in the art (see, for example, Remington: the Science and Practice of Pharmacy, ISBN 0857110624).

As used herein, the term *treating* or *treatment* of any cancer refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

### Methods to obtain a neopeptide sequence

A first aspect of the invention relates to a method to obtain an immunogenic variant of a candidate peptide sequence comprised in a tumour antigen polypeptide expressed in a cancer patient, or commonly expressed in patients with a certain class of cancer. The resulting variant peptide sequence is referred herein as a neopeptide sequence, and may be of use, for example, in an *in silico* library of neopeptide sequences from which a clinician may select suitably HLA-restricted options for administration to a recently diagnosed cancer patient. Alternatively, the method may be applied to epitope comprised within protein sequences encoded by a specific patient's tumour genetic sequencing result, to create a personalised library of neopeptide sequences. In particular embodiments, a candidate peptide sequence is a T cell epitope restricted to an HLA commonly present in the population, for example, expressed at more than 1% frequency at the population level. In other particular embodiments, it is restricted to an HLA allele in a certain individual for whom a peptide component of a personalised cancer vaccine is being designed.

The method entails identifying, or providing a candidate peptide sequence 8 to 25 amino acids in length with characteristics ensuring it is suitable as a T cell epitope (an MHC-restricted peptide) in the context of cancer vaccination. A candidate peptide sequence according to the invention has a certain length, and comprises a conserved amino acid motif that confers binding to an HLA class I or an HLA class II molecule, and presentation to T cells. The candidate peptide sequence is comprised in the primary amino acid sequence of a tumour antigen expressed in the patient, or expressed the patient's specific cancer tissue subtype, for example a protein often expressed in a glioblastoma tumour. In some embodiments, the candidate peptide sequence may be a known T cell epitope associated with T cell responses in the patient's class of cancer, derived from studies of immunopeptidomes or immunodominance studies investigating the roles of T cell epitopes in cancer immunity. In alternative embodiments, a tumour antigen may be a protein sequence commonly expressed in tumour tissue samples of the patient's specific cancer class.

In particular embodiments of the identification step according to the invention, the method includes identifying personalised tumour antigens expressed (at the level of gene or protein expression) in a tumour sample obtained from a cancer patient. Means of identifying particularly desirable personalised tumour antigens are addressed in detail in the section entitled *Candidate peptide sequence identification.* In brief, to obtain a candidate peptide sequence, the protein sequence of a tumour antigen expressed in a patient, and a target HLA-allele are inputted into a T cell epitope prediction algorithm (e.g. an online tool such as NET-MHCpan 4.0), to provide a plurality of candidate peptide sequences predicted to bind specifically to the HLA allele of the patient.

A candidate peptide sequence according to the first aspect of the invention comprises an HLA-binding motif 8 to 11 amino acids in length, comprising conserved amino acids at specific positions that confer specific binding to an HLA molecule. Most HLA-binding motifs are between 8 and 11 amino acids long. In particular embodiments, the candidate peptide sequence is an HLA class I-binding motif 9 amino motif. In some embodiments, the candidate peptide sequence consists only of the HLA-binding motif, without any additional flanking amino acids. A candidate peptide sequence may comprise more than one HLA-binding motifs, if it is an HLA class II restricted peptide with multiple HLA-binding registers. An HLA-binding motif is a region of 8-11 amino acids, of which 3 to 5 amino acids are conserved among T cell epitopes known to bind to the selected HLA allele. An HLA-binding motif is identified in a candidate peptide sequence with reference to a specific HLA allele present in the patient. Such a motif may be identified, for example, by means of a T cell epitope prediction algorithm such as publicly available only tools such as NET-MHC, or SYFPEITHI.

In order to induce desirable T cell activation, a candidate peptide sequence according to this aspect of the invention binds specifically with a peptide-binding groove of either an HLA class I allele, or an HLA class II allele expressed by the patient. The HLA allele type of a cancer patient may be assessed by clinical HLA-typing assays known in the art, particularly high-resolution HLA gene sequencing of a DNA sample obtained from the patient.

In an *in silico* HLA-anchor assignment step, the method entails inputting the candidate peptide sequence into an HLA-anchor prediction algorithm in order to identify the conserved amino acid residues that form close contacts between the candidate peptide sequence, and a particular HLA class I or class II allele present in the patient. An HLA-anchor prediction algorithm accepts as an input a peptide sequence, and an identity of an HLA class I or an HLA class II allele. The HLA-anchor prediction algorithm compares a candidate peptide sequence to a database of known conserved amino acid motifs that characterise all peptides which bind to the specified inputted patient HLA allele, and assigns these residues as HLA-anchor amino acids. For each of the one or more HLA-binding motifs located within the candidate peptide sequence (regions of 8-11 amino acids), the algorithm identifies between 3 to 5 amino acids conserved among T cell epitopes known to bind to the selected HLA allele, and assigns an identity to these essential contact residues as HLA-anchor amino acids. In particular embodiments, this process may be performed simultaneously for a plurality of candidate peptide sequences, by inputting a full tumour antigen protein sequence, rather than a candidate peptide sequence. In such embodiments, an tumour antigen protein sequence is broken down *in silico* into a plurality of T cell epitopes overlapping by one amino acid - each a potential candidate peptide sequence - and each candidate is assigned a ranking of their predicted binding strength (strong, weak, no binding) in reference to the patient's HLA allele. Both strong and weak binders generate functional neopeptides according to the invention.

For most HLA class II and class I alleles, the N-terminal amino acid of the HLA-binding motif comprised within a candidate peptide sequence, and the adjacent amino acid, and the C-terminal amino acid of the HLA-binding motif, and the adjacent amino acid are conserved amino acid residues. The inventors predict that amino acid substation placed at these 4 residues may be more likely to interrupt the specific interaction with HLA facilitating antigen presentation to T cells. Thus, in particular embodiments, the N-terminal amino acid of the HLA-binding motif, and the adjacent amino acid, and the C-terminal amino acid of the HLA-binding motif, and the adjacent amino acid are assigned as HLA-anchors according to the invention, even if not assigned as HLA-anchor amino acids by an HLA-anchor prediction algorithm according to the invention, (for example SYFPEITHI). Remaining amino acid residues available for substitution, not assigned HLA-anchor amino acids according to the invention are sometimes referred to in this specification as TCR-anchors, as they are predicted to mediate the affinity and avidity of binding to T cell receptors.

A next step of the method according to the first aspect of the invention is an *in silico* neopeptide design step. Here, at least one, or two amino acid substitutions are introduced into the candidate peptide sequence, to provide a new peptide sequence, encoding a variant peptide referred to herein as a neopeptide. At least one amino acid substitution is placed within the HLA-binding motif. Positions outside the HLA-binding motif are less critical for HLA-binding and thus may be targeted by further additions, deletions, or substitutions. To ensure the introduction of these amino acid substitutions does not disturb the ability of the peptide to bind to the HLA class I or class II allele of the patient and be presented to T cells, each of the one or more substitutions is introduced at a position within the HLA-binding motif which has not been assigned as an HLA-anchor amino acid in the previous step.

The invention delivers a neopeptide sequence encoding a neopeptide sufficiently different from the naturally candidate peptide sequence expressed by the patient's tumour to appear "foreign" to a patient's immune system when presented to T cells in the context of the patient's MHC molecules on the surface of cells. The targeted position, and limited number of the amino acid substitutions ensures that neopeptides retain their important ability to binding to the HLA-binding groove of an HLA class I or class II molecule.

In some embodiments, the method further comprises synthesising the neopeptide sequence to provide a neopeptide. Such a neopeptide may be of use for screening assays to search for T cell epitopes associated with certain desirable T cell effector responses, such as cytokine production, or tumour cell cytotoxicity, or for incorporation into a library of peptides available for vaccine compositions.

### Neopeptides according to the invention

Another aspect of the invention is a neopeptide molecule. The neopeptide is characterised by a variant peptide sequence differing by one, or two amino acids substitutions compared to a candidate peptide sequence, at least one of which is placed within an HLA-binding motif. Positions outside the HLA-binding motif are less critical for HLA-binding and thus may be targeted by further additions, deletions, or substitutions. A candidate peptide sequence is an MHC-restricted T cell epitope peptide comprised in the primary amino acid sequence of a tumour antigen, a protein expressed in cancer cells (see *Candidate peptide sequence identification*). In particular embodiments, the neopeptide is derived from candidate peptide sequence within a tumour protein expressed in a tumour sample obtained from a cancer patient.

A neopeptide according to this aspect of the invention comprises within its peptide sequence an HLA-binding motif 8 to 11 amino acids in length. In particular embodiments, the length of the internal HLA-binding motif is 9 amino acids. The length of the neopeptide itself may be longer, particularly from 8 to 10 amino acids for HLA class I-restricted peptides, or 8 to 25 amino acids for HLA class II restricted peptides. In embodiments particularly relevant to HLA class I-binding neopeptides, the neopeptide consists of the HLA-binding motif and no further flanking peptides. HLA class II-binding neopeptides are often longer, comprising additional flanking amino acid residues outside the HLA-binding motif. An HLA-binding motif within the neopeptide according to this aspect of the invention interacts specifically with a peptide-binding groove of a human HLA class I allele, or a human HLA class II allele. In particular embodiments wherein the neopeptide is part of a personalised vaccine strategy, the HLA-binding motif interacts specifically with an HLA class I allele, or a HLA class II allele expressed in a cancer patient, as determined by a HLA-typing assay.

When comparing a naturally-occurring candidate peptide sequence (from a tumour-expressed protein), with the sequence of a neopeptide, the central amino acids of the HLA-binding motif are characterised by one, or two amino acids substitutions, with the proviso that when the candidate peptide sequence is inputted into an HLA-anchor prediction algorithm configured to assign an amino acid required for specific association of the HLA-binding motif to the HLA class I allele or the HLA class II allele present in the cancer patient as an HLA-anchor amino acid, the one or two amino acids at the position within the HLA-binding motif corresponding with the one, or two substituted amino acids are not assigned as an HLA-anchor amino acid. In other words, in comparison to a candidate peptide sequence, the neopeptide is characterised by 1, or 2 amino acids exchanged at amino acid positions within the HLA-binding motif, and between, or adjacent to HLA-anchor amino acids.

For a neopeptide comprising an HLA-binding motif characterised by an even number of amino acids, at least one of the amino acids substitutions is one of the 2, 4 or 6 central amino acids of the HLA-binding motif. In particular embodiments, at least one of the amino acids substitutions is one of the 4 central amino acids of the HLA-binding motif. In particular embodiments, at least one of the amino acids substitutions is one of the 2 central amino acids of the HLA-binding motif. For a neopeptide comprising an HLA-binding motif characterised by an odd number of amino acids, at least one of the amino acids substitutions is one of the 5 central amino acids of the HLA-binding motif. In particular embodiments, at least one of the amino acids substitutions is one of the 3 central amino acids of the HLA-binding motif.

As cancer vaccination protocols may comprise repeated administration of a neopeptide according to the invention, in some embodiments of a neopeptide, an immunogenicity screening may be carried out on a patient sample to identify neopeptides associated with an immune response for continued application to the patient. The inventors found that T cell activation assays carried out on tumour infiltrating lymphocytes (TIL) obtained from patient tumour tissue sample can detect immune responses generated in response to vaccination with neopeptides. In particular embodiments of a neopeptide according to the invention, wherein the patient has received a vaccination comprising the neopeptide according to the invention in the month prior to tissue sampling, contacting a cell preparation comprising CD4+ T cell and/or CD8+ T cells isolated from a tumour sample obtained from the cancer patient with the neopeptide evokes proliferation of the CD4+ T cell or CD8+ T cells. Concentrations of IFN-*γ* or other proinflammatory cytokines in the supernatant after stimulation, or expression levels of activation markers CD69 and CD25, or the cytotoxicity marker Granzyme B on CD4+ T cells are all examples of, but not limited to, markers associated with neopeptide stimulation according to the invention. In particular embodiments, a neopeptide according to the invention is characterised by a capacity to induce proliferation of reactive T cells after stimulation with the neopeptide *in vitro,* for example, using a method such as CFSE dilution, or radioactive thymidine incorporation. In particular embodiments, the following assay is used to determine a level of cell proliferation. In brief, T cells isolated from a patient tumour sample (e.g. biopsy material, or tumour material from a resection procedure carried out on a patient vaccinated with a neopeptide within the 3 months prior to sample collection) are stimulated with 5 of the neopeptide for 5 days, and proliferation is measured by ³H-thymidine incorporation assay. For peptide stimulation assay of TILs, TILs are seeded at 5 × 10⁴ cells/well with 2 × 10⁵ irradiated autologous PBMCs (45Gy) as APCs in 200 µl X-VIVO 15^{™} medium (Lonza) in 96-well U-bottom plates and stimulated with peptides at a final concentration of 5 µM. Proliferation is measured at day 5 by ³H- incorporation assay. The proliferation strength is depicted as a stimulation index (SI). SI indicates the ratio of counts per minute (cpm) generated by incorporated ³H-thymidine in the presence of the peptide versus cpm of the no peptide control. An SI of 2 or above characterises a neopeptide according to the invention with the capacity to recruit, and activate CD4 or CD8 T cell clones within the tumour.

### Neopeptide sequences according to the invention

In some embodiments of either the method according to the first aspect of the invention, or the neopeptide as specified in the second aspect described above, two amino acid substitutions characterise the amino acid sequence of the neopeptide, when it is compared to the candidate peptide sequence. In particular embodiments, a single amino acid differs between the candidate peptide sequence and the amino acid sequence of the neopeptide sequence. In still more particular embodiments, a single substitution is present next to one of the 4 central amino acids of an even-numbered HLA-binding motif, or the 5 central amino acids of an odd-numbered HLA-binding motif. Certain embodiments are relevant to neopeptides generated to stimulate CD8+ T cells. In some embodiments of the method to obtain a neopeptide sequence, or the neopeptide according to the invention, the HLA-binding motif of the neopeptide sequence (and thus the candidate peptide sequence, the patient-derived T cell epitope/predicted T cell epitope from which it is derived) is a motif which interacts specifically with an HLA class I polypeptide. Accordingly, the neopeptide sequence has characteristics favouring binding to HLA class I molecules, namely a length of between 8 and 11 amino acids. In particular embodiments relating to HLA class I restricted peptides, the neopeptide sequence consists of 9 amino acids.

Alternative embodiments relate to neopeptides of use to stimulate CD4+ T cell responses. In these embodiments of the method or neopeptide according to the invention, each HLA-binding motif of the neopeptide sequence (and thus the candidate peptide sequence, the T cell epitope or predicted T cell epitope from which it is derived) is a motif which interacts specifically with an HLA class II polypeptide. Accordingly, the neopeptide sequence has characteristics favouring binding to HLA class II molecules, namely a length of between 8 and 25 amino acids. In particular embodiments relating to HLA class II restricted peptides, the neopeptide sequence consists of 11 amino acids. In other particular embodiments relating to HLA class II restricted peptides, the neopeptide sequence consists of 12 amino acids. In further embodiments relating to HLA class II restricted peptides, the neopeptide sequence consists of 13 amino acids.

The data presented in the examples shows that both conservative and non-conservative amino acid changes can generate neopeptides with the capacity to efficiently stimulate T cell responses.

In certain embodiments of the method to obtain a neopeptide sequence, or the neopeptide according to the invention, the amino acid substitution differentiating the sequence from the tumour T cell epitope on which it is based, is a conservative amino acid substitution. In other words, an amino acid with similar physiochemical properties replaces the original residue. In particular embodiments, the substitution is a conservative amino acid change selected from the following list:
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable;
- methionine (M) and Q are interchangeable;
- cysteine (C), A and S are interchangeable;
- proline (P), G and A are interchangeable;
- arginine (R) and lysine (K) are interchangeable.
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable; N and D are interchangeable; E and Q are interchangeable;
- methionine (M) and Q are interchangeable;
- cysteine (C), A and S are interchangeable;
- G and A are interchangeable.

In alternative embodiments of the method to obtain a neopeptide sequence, or the neopeptide characterised by a neopeptide sequence, the substitution that differentiates the neopeptide from the human tumour protein epitope on which it is based is a non-conservative amino acid substitution, not listed in the paragraph above. A non-conservative amino acid change is one in which the charge, or the side chain structure, hydrophobicity or size are different or one that causes a structural change such as a "kink" in the peptide caused by introduction of a Proline (P).

In some embodiments in addition to a centrally placed amino acid substitution to alter the nature of the interaction with the TCR, the neopeptide is additionally characterised by an asparagine to lysine replacement at the N-terminus outside the core interphase between TCR and HLA/peptide, to increase the solubility of the peptide. In other embodiments, the neopeptide is additionally characterised by a substitution of a D-alanine to increase peptide stability. It is well-known that post-translationally modified amino acids, e.g. citrulline as a modification of arginine, are targets in autoimmune diseases such as rheumatoid arthritis. Accordingly, in certain embodiments a neopeptide, or peptide characterised by a neopeptide sequence obtained according to the invention includes a post-translational modification to render the peptide more immunogenic, particularly located outside the HLA-binding motif.

In particular embodiments of the method to obtain a neopeptide sequence, or the neopeptide according to the invention, the candidate peptide sequence on which the neopeptide is based is characterised by a predicted level of specific binding IC50 value of at least 500nM to a HLA class I molecule.

In particular embodiments of the method to obtain a neopeptide sequence, or the neopeptide according to the invention, the candidate peptide sequence on which the neopeptide is based, is characterised by a predicted level of specific binding IC50 value of at least 1000nM to a HLA class II molecule.

### Candidate peptide sequence identification

The first aspect of the invention above describes how neopeptides according to the current invention are obtained from candidate peptide sequences with specific physiological characteristics such as a prescribed length, and HLA-binding motifs. These features ensure the resulting neopeptide specifically interacts with (binds to), the antigen-binding groove of an HLA molecule present in the patient for whom a vaccine treatment is designed, in order to facilitate presentation of the neopeptide to CD4+ or CD8+ T cells. Further characteristics relating to the expression levels in tumours of candidate peptide sequences according to the invention are described below.

Highly immunogenic neopeptides that are recognized in the context of frequent HLA class I and II alleles and look promising in several patients, can be used to generate a peptide warehouse useful as a rapidly available medicament while the process of personalised medicine development takes place. In certain embodiments of the method to obtain a neopeptide sequence, or the neopeptide according to the invention, the amino acid sequence of neopeptides is derived from a candidate peptide sequence in a tumour antigen present in a cohort or patients characterised by a certain type of cancer, for example a T cell epitope commonly expressed in more than 50%, 80% or 90% of patients with a type of cancer. This embodiment is relevant to a library, or database of neopeptides, from which a clinician may select a neopeptide (or a neopeptide sequence for subsequent synthesis) for clinical administration, which likely to be expressed in a type of cancer matching that diagnosed in a patient. Particularly, a clinician may select several neopeptides targeting a range of tumour antigens, and a plurality of HLA alleles expressed by the patient (See *Vaccine compositions comprising neopeptides*). A candidate peptide sequence according to this embodiment is characterised by a mean expression level in a plurality of tumour samples obtained from a plurality of cancer patients is at least 1.5 folder greater than a mean expression level of a plurality of matched healthy tissue samples obtained from a plurality of healthy individuals. Such a candidate peptide sequence is referred to a tumour associated antigen in the study terminology in the examples, and is an antigen demonstrated, or predicted to be expressed or immunologically important in the cancer. In particular embodiments, the patient has been diagnosed with glioblastoma, and the neopeptide is one listed in Table 1 of the examples, comprising 12 TAAs reported in the context of glioblastoma based on immunopeptidome- or sequencing analyses.

Alternatively, the method or neopeptide may be part of a personalised vaccine treatment design strategy, where the candidate peptide sequence is matched to both the patient's own HLA-type, and the specific antigen expression profile of the patient's own tumour. In particular embodiments of the method to obtain a neopeptide sequence, or the neopeptide according to the invention, the candidate peptide sequence is present within a protein expressed in the tumour sample obtained from the cancer patient. Means of measuring the expression of antigens in a tumour sample acquired from a cancer patient are not particularly limited, including mRNA expression profiling, proteomics profiling, and/or immunopeptidome profiling. In particular embodiments, the candidate peptide sequence is comprised within a tumour antigen protein sequence encoded by a gene with an mRNA expression level in a tumour sample obtained from the patient exceeding a threshold level for high expression. The threshold may be a number of reads of the gene in question, particularly more than 20 reads. In more particular embodiments, the mRNA encoding the tumour antigen is characterised by over 100 reads per million. Still particularly, more than 1000 reads per million. In more particular embodiments, the mRNA expression of a gene encoding the tumour antigen comprising the candidate peptide sequence is at, or above a threshold of the mean, or median of a cohort gene expression distribution obtained from a plurality of tumour samples matching the tumour type of the patient. In further particular embodiments, the threshold for gene expression greater than that is considered overexpression is at, or above a threshold of 1.5 times the interquartile range greater than the 75th percentile in a cohort gene expression distribution obtained from a plurality of tumour samples matching the tumour type of the patient.

In certain embodiments, the candidate peptide sequence comprises a tumour specific, somatic mutation (non-inherited, sporadically arising), in other words, a mutation present within tumour cells, but not healthy cells obtained from said patient. Somatic mutations may be determined by DNA, or mRNA sequencing of tumour, and comparison to a non-tumour sample, for example, genetic material obtained from a peripheral blood sample. A somatic mutation at the DNA level may be a nucleotide exchange that is silent with respect to amino acid sequence, and thus does not create a novel peptide with immunogenic potential. Desirable somatic mutations according to the invention, are those that generate non-silent mutations that result from alternative splicing, single amino acid mutations, or other specific DNA changes that lead to an altered protein sequence. T cell epitopes comprising such non-silent somatic mutations are useful candidate peptide sequences according to the invention, and may be transformed into a neopeptides or neopeptide sequences by the addition of further, artificial amino acid substitutions in addition to the somatic mutation identified in the patient tumour sample. The somatic mutation is not the site of an amino acid substitution, it is conserved in the neopeptide sequence. In particular embodiments, a somatic mutation identified in a whole-exome sequencing (WES) sample of tumour tissue and not present in a peripheral blood mononuclear cells (PBMCs) characterises the candidate peptide sequence, and the related neopeptide sequence according to the invention.

In certain embodiments, the candidate peptide sequence is comprised within a cancer-associated testis protein antigen sequence, as these have been shown to be associated with tumour malignancy and progression, making them desirable targets for interventions that increase immune responses. In particular embodiments, the candidate peptide sequence is comprised within the New York oesophageal squamous cell carcinoma -1 (NY-ESO-1, UniProt P78358) protein sequence. In other particular embodiments, the candidate peptide sequence is comprised within the melanoma-associated antigen 1 (MAGE-A1, Uniprot P43355) protein sequence. In other particular embodiments, the candidate peptide sequence is comprised within the protein sequence of a member of the synovial sarcoma X breakpoint (SSX) family of testis antigens.

In particular embodiments, the candidate peptide sequence is comprised within a protein antigen associated with an immunologically cold solid tumour type. Immunologically cold tumours are can be identified by a clinician by an absence of immune infiltrate, as determined by pathology analysis of a histological section obtained from a patient's tumour. More particularly, an immunologically cold tumour is characterised by an absence or paucity of CD3+ T cells as determined by histological pathology analysis. A common threshold applied to define such an immune cold tumour is fewer than 300 T cells identified in microscopic analysis of a histological section obtained from a patient tumour stained with labelled antibody specific for the T cell marker as CD3. This definition is to be employed to define the term "immunologically cold tumour" as used herein.

In other particular embodiments, the cancer is characterised by a tumour with a low mutation burden. Tumour mutation burden is defined as the number of non-inherited, somatic mutations per million bases of an investigated genomic sequence, either of a cohort of patients diagnosed with the same tumour type, or a representative tumour sample, or a particular cancer patient for which a personalised vaccine is being developed. A low mutation burden according to current clinically standards is 10 mutations per million bases, as defined by the U.S Food and Drug Administration as threshold for use for prescribing the checkpoint inhibitor drug pembrolizumab. This definition is to be employed to define the term "a tumour of low mutation burden" as used herein.

In certain embodiments the candidate peptide sequence is comprised with a protein expressed in the tumour of a patient diagnosed with pancreatic cancer.

In other embodiments the candidate peptide sequence is comprised with a protein expressed in the tumour of a patient diagnosed with prostate cancer.

In other embodiments the candidate peptide sequence is comprised with a protein expressed in the tumour of a patient diagnosed with neuroblastoma.

In other embodiments the candidate peptide sequence is comprised with a protein expressed in the tumour of a patient diagnosed with glioma.

### Vaccine compositions comprising neopeptides

Another aspect of the invention relates to a vaccine composition comprising a neopeptide according to the second aspect of the invention, or a peptide characterised by a neopeptide sequence obtained as specified in the first aspect of the invention. In particular embodiments, the vaccine composition comprises a plurality of neopeptides according to the invention, wherein at least one in an HLA class I restricted peptide, and at least one is an HLA class II-restricted peptide. In more particular embodiments, the vaccine composition comprises 10 to 30 neopeptides according to the invention, wherein approximately half comprise an HLA class I HLA-binding motif, and about half comprise an HLA class II HLA-binding motif.

A vaccine composition according to the invention may further comprise a T cell adjuvant. In particular embodiments, the vaccine composition comprises Imiquimod. In other embodiments, the composition comprises XS15 (pam3CYs conjugated to a peptide) and Montanide ISA 51 (a water in oil emulsion composed of a mineral oil and a surfactant from the mannide monooleate family), a combination proposed to activate cytotoxic T cell responses).

In particular embodiments, the vaccine composition comprises a plurality of neopeptides predicted to bind specifically to the HLA alleles expressed by a cancer patient for whom the composition is designed. In further particular embodiments, the vaccine composition comprises 4 to 30 neopeptides, wherein at least one is predicted to bind to at each of at least 4 HLA alleles expressed by the cancer patient. In still more particular embodiments, the vaccine composition comprises 10 to 30 neopeptides, wherein at least one is predicted to bind to each of the 6 HLA alleles expressed by the cancer patient.

In particular embodiments of the vaccine composition according to the invention, it comprises a plurality of neopeptides according to different embodiments of the invention described above. Preferably, it combines neopeptides based on candidate peptide sequences overexpressed in a tumour sample obtained from the patient, optionally, some further characterised by somatic mutations, and neopeptides based on candidate peptide sequences comprised within tumour antigens associated with immunodominant anti-cancer responses to the patient's particular tumour type. Optionally, the vaccine composition further comprises peptides without amino acid substitutions, corresponding to original, naturally occurring candidate peptide sequences.

### Nucleic acid expression vectors encoding neopeptides

Another aspect of the invention relates to nucleic acid vectors encoding a neopeptide sequence, particularly a neopeptide sequence obtained by a method as specified in the section entitled *Methods to obtain a neopeptide sequence.* RNA-based vaccination can provide a flexible and affordable personalised medicament. Methods for generating such personalised mRNA vaccines are well known, and may be used to administer vectors encoding a neopeptide sequence according to the invention (Salomon N., Oncoimmunology 2020, 9:1771925; Sahin U., Nature 2017, 547:222). The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell. Such a nucleic acid expression vector may be of use as a vaccine driving transcription, and translation of a neopeptide according to the invention in a patient diagnosed with cancer.

### Pharmaceutical Compositions, Administration/Dosage Forms and Salts

A next aspect of the invention relates to a pharmaceutical composition comprising a neopeptide, or a neopeptide sequence obtained by a method as specified above in *Methods to obtain a neopeptide sequence,* or a nucleic acid expression vector as described in the previous paragraph, for use in treating cancer.

In particular embodiments of the pharmaceutical composition the cancer is an immunologically cold tumour, wherein a tumour sample is characterised by an absence, or a paucity of T cells. In other particular embodiments, the pharmaceutical composition is provided for treating a patient diagnosed with a tumour type with a low mutation burden.

In other particular embodiments, the pharmaceutical composition is provided for use in a patient diagnosed with pancreatic cancer. In other embodiments the candidate peptide sequence is comprised with a protein expressed in the tumour of a patient diagnosed with prostate cancer.

In other embodiments the pharmaceutical composition is provided for use in a patient diagnosed with neuroblastoma. In other embodiments the pharmaceutical composition is provided for use in a patient diagnosed with glioma.

In particular embodiments, the pharmaceutical composition is provided for use in a use in a patient not receiving dexamethasone, which undesirably dampen vaccine immune responses.

In particular embodiments, the pharmaceutical composition is provided for use in a use in a patient receiving a checkpoint inhibitor antibody medicament, particularly and an anti-CTLA-4, anti-PD-1 or anti-PD-L1 antibody.

According to one aspect of the neopeptide according to the invention, the neopeptide according to the invention is provided as a pharmaceutical composition, pharmaceutical administration form, or pharmaceutical dosage form.

The skilled person is aware that any specifically mentioned neopeptide mentioned herein may be present as a pharmaceutically acceptable salt. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

In certain embodiments of the invention, the compound of the present invention is typically formulated into pharmaceutical dosage forms to provide an easily controllable dosage of the drug and to give the patient an elegant and easily handled product.

Similarly, a dosage form for the prevention of recurrence or treatment of cancer is provided, comprising a tumour neopeptide, or a vaccine preparation comprising a tumour neopeptide, or a nucleic acid vector encoding a neopeptide according to any of the above aspects or embodiments of the invention.

In particular embodiments, a neopeptide, or a plurality of neopeptides are delivered as monthly injections as tested in the examples. In other particular embodiments, the neopeptide is administered weekly. The concentration of peptide within each injection is preferably in the range of 25 µg to 50 mg. In particular embodiments a neopeptide dose according to the invention is delivered in a dose of 25 µg (class I), or 200 µg (class II)/peptide).

The invention further encompasses a pharmaceutical composition comprising a neopeptide of the present invention, or a pharmaceutically acceptable salt thereof, and a pharmaceutically acceptable carrier. In further embodiments, the composition comprises at least two pharmaceutically acceptable carriers, such as those described herein.

Certain embodiments of the invention relate to a dosage form for parenteral administration, such as subcutaneous, intravenous, injection into the vicinity of a tumour, intrahepatic or intramuscular injection forms. Optionally, a pharmaceutically acceptable carrier and/or excipient may be present.

The dosage regimen for the neopeptides of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the neopeptides of the invention may be administered in a single daily dose, or the total daily dosage may be administered in divided doses of two, three, or four times daily.

The therapeutically effective dosage of a neopeptides, nucleic acid expression vector, the pharmaceutical composition, or combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc.. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

Multiple aspects or embodiments of the invention may be employed in a multi-pronged approach applicable to personalized tumour vaccination to more patients and a broader range of cancers. Several prerequisites would need to be met. HLA genotyping, target predictions and choices of peptides including the design of the neopeptides would need to be performed rapidly in patients with glioblastoma, pancreatic cancer, or other tumours with rapid progression. Furthermore, in contrast to the current trial, where non-GMP peptides were applied under the regulation of an individual treatment attempt, GMP peptides or nucleic acid vectors need to be synthesized rapidly, and an acceptable cost in order to accommodate clinical trials with larger patient numbers. Patients could then first be vaccinated with warehouse peptides and in the successive rounds of vaccination with a combination of pre-made peptides and those that are specifically designed for the patient.

In some embodiments, a vaccine composition or pharmaceutical composition according to the invention is administered to a patient in combination with a checkpoint inhibitor antibody. In particular embodiments the vaccine is administered in combination with an anti-PD-1 or PD-L1 antibody.

In some embodiments, a vaccine composition or pharmaceutical composition according to the invention is administered to a patient in concurrently (within a medically relevant window of 2 months) receiving radiochemotherapy.

The invention further encompasses, as an additional aspect, the use of a neopeptide, or a vaccine composition as identified herein, or its pharmaceutically acceptable salt, as specified in detail above, for use in a method of manufacture of a medicament for the treatment or prevention of a condition cancer. In more particular embodiments, neopeptides are used in the manufacture of a medicament to treat a cancer selected from prostate cancer, pancreatic cancer, or brain cancer, particularly glioma. Similarly, the invention encompasses methods of treatment of a patient having been diagnosed with a disease associated with a tumour characterised by a mutation burden of less than 10 mutations per million bases. This method entails administering to the patient an effective amount of a neopeptide, or a vaccine composition as identified herein according to the above aspects of the invention, or its pharmaceutically acceptable salt, as specified in detail herein.

The invention further encompasses the use of:
- HLA-typing nucleic acid probes and assay reagents,
- mRNA sequencing reagents,
- software which can identify tumour antigens with a high mRNA expression level in the patient's tumour sample by applying a threshold to each gene, and further for identifying candidate peptide sequences within the proteins encoded by said highly expressed tumour antigens, and performing *in silico* targeted mutation of the central amino acids of said candidate peptides to deliver a list of putative neopeptide sequences that may be synthesised, or incorporated into nucleic acids vectors to create a personalised cancer vaccine.
for use in the manufacture of a kit for the detection of candidate peptide sequences, to obtain a neopeptide sequence according to the invention.

### Computer methods

Methods to obtain a neopeptide sequence, or neopeptide according to the invention may comprise, or be embodied by way of a computer-implemented method, particularly wherein the measurement of tumour antigen expression, the generation of candidate peptides from tumour antigens, the generation of in silico sequences comprising the amino acid substitutions in tumour neopeptides, the evaluation of candidate peptide predicted binding to HLA alleles, or the assignment of certain amino acid residues as HLA-anchors, are executed by a computer. Further, the method may be embodied by way of a computer program, comprising computer program code, that when executed on the computer cause the computer is designed and configured to execute at least the evaluation and/or assignment steps. Particularly, the results of the tumour antigen expression measurements, such as an mRNA sequencing assay, may be provided to the computer and/or the computer program by way of a user input and/or by providing a computer-readable file comprising information regarding the expression level obtained during the measurement step. Results from the computer implemented steps may be stored for further processing on a memory of the computer, on a non-transitory storage medium.

### Combinations of aspects and embodiments of the invention

Wherever alternatives for single separable features such as, for example, a certain neopeptide sequence, or length, or cancer type indication are laid out herein as "embodiments", it is to be understood that such alternatives may be combined freely to form discrete embodiments of the invention disclosed herein. Thus, any of the alternative embodiments for a neopeptide may be combined with any of the alternative embodiments of an *in silico* neopeptide sequence, a synthesised neopeptide, or vaccine composition and these combinations may be combined with any medical indication or diagnostic method mentioned herein.

To give one example of the method according to the invention incorporating some of the embodiments described above and illustrated in Fig. 1, a patient blood sample is subjected to HLA-typing, showing the patient expresses the HLA A* 26 01 allele. A patient tumour sample is obtained, and subjected to mRNA sequencing, showing high expression of a tumour antigen X. Submitting the HLA-type, and protein sequence of protein X to the NET-MHCpan 4.0, a candidate peptide sequence Y is identified. Inputting the peptide sequence of Y and HLAA* 26 01 into the database of SYFPEITHI identifies putative HLA-binding motifs comprising preferred amino acids present in peptide anchor positions 2, 6 and 9. Positions 1, 2, 8, and 9 are assigned as HLA-anchors according to the invention due to their position at either end of the HLA-binding motif, and/or because they are assigned HLA-anchor amino acids by SYFPEITHI, as well as 6, which is a conserved HLA-anchor assigned by SYFPEITHI for HLA A* 26 01. As a result, a substitution to obtain a neopeptide according to the invention may be at one, or two of, positions remaining, either 3, 4 and 5. As centrally positioned amino acid substitutions are preferred, thus, an amino acid substitution is introduced at position 5, to generate a neopeptide sequence according to the invention. This neopeptide sequence is synthesised as a neopeptide, and administered to the patient in a vaccine composition at an effective dose. The following month, a tumour biopsy is performed. It is confirmed that this neopeptide is associated with CD8 T cell division *in vitro,* when applied to an *in vitro* white blood cell sample obtained from the patient tumour biopsy sample. Further doses of the personalised vaccine comprising the neopeptide are then administered to the patient.

The invention further encompasses a method such as the following method steps for generating a plurality of designed tumour neopeptides for use in personalise cancer vaccine for treating a patient diagnosed with a malignant neoplastic disease, particularly glioma.

In a tumour antigen identification step, obtaining a plurality of tumour antigen protein sequences characterised by an expression level of the gene encoding the tumour antigen protein sequence:
- exceeding a threshold expression level in a tumour obtained from the patient; and/or
- exceeding a threshold expression level in a cohort of tumour samples from patients diagnosed with the same cancer as that patient, in comparison to a cohort of matched tissue samples of healthy individuals.

In an HLA-typing step, obtaining the HLA phenotype of the patient to provide a plurality of HLA class I (e.g. HLA-A, and HLA-B alleles as shown in the study) and HLA class II (particularly those assessed in the study, DRB1, DRB3, DRB4, DRB5, DPA1, DPB1, DQA1, and DQB1) alleles present in the patient/patient sample;.

In an *in silico* candidate peptide prediction step, submitting the tumour antigen protein sequence to an HLA-binding prediction algorithm. In brief, the HLA genotype of the patient, and each tumour antigen is inputted into a T cell epitope prediction software (particularly NetMHCpan 4.0(46)) and the MHC-I and MHC-II binding prediction tools (IEDB recommended mode) of the IEDB Analysis Resource are suited to obtain an output of
- a plurality of candidate peptide sequences comprising an amino acid sequence 8-11, particularly 9 amino acids in length all predicted to have weak, or strong binding to an HLA class I allele identified in the HLA-typing step, and
- a plurality of candidate peptide sequences comprising 10 - 25 amino acids, particular 11, 12, or 13 amino acids long, all predicted to have weak, or strong binding to an HLA class II allele identified in the HLA-typing step.

All resulting candidate peptide sequences are assigned HLA-anchor amino acids using software such as SYFPEITHI, in brief each candidate sequence and its associated HLA allele are imputed into SYFPEITHI to:
- identity amino acid residues which comprise at least one HLA-binding motif within each candidate peptides conferring specific binding to a HLA peptide binding groove of the HLA class I alleles or HLA class II expressed by the patient, and assigning them as HLA-anchor amino acids.

In a neopeptide design step, introducing into each of the plurality of candidate peptides one amino acid substitution at an amino acid which is **not:**
- the N-terminal, or the C-terminal amino acid of the candidate peptide, or the amino acid adjacent to the N-terminal, or the C-terminal amino acid of the candidate peptide, or
- an amino acid assigned as an HLA-anchor amino acid in the HLA-anchor assignment step;
   particularly wherein the amino acid substitution is introduced at the central amino acid, or one of the two central amino acids of the HLA-binding motif;
to provide a plurality of neopeptide sequences.

Selecting from the resulting *in silico* neopeptide sequence library at least one neopeptide predicted to bind to each of the patient's HLA, particularly 30 amino acids in total derived from a range of tumour antigen proteins present in the patient, and synthesising them for use in a vaccine composition. In particular embodiments of the method, roughly half of the peptides synthesised for inclusion in the vaccine composition are MHC class II restricted, and the other half are MHC class I restricted.

In an optional patient optimisation step following administration of the vaccine composition in the preceding paragraph, T cells from a patient tumour sample are screened for reactivity to the neopeptides sequences failing to induce T cell proliferation in a thymidine incorporation assay, are replaced by alternative neopeptides synthesised from the *in silico* neopeptide sequence library.

The invention is further illustrated by the following examples and figures, from which further embodiments and advantages can be drawn. These examples are meant to illustrate the invention but not to limit its scope.

### Description of the Figures

- Fig. 1: shows strategy for predicting TSAΓΓAA derived peptides and for designing D neopeptides by mutations of peptide to putative TCR contact positions. (A) Cells of the primary glioblastoma and PBMCs were collected for WES to identify somatic mutants in the tumour, which were considered as TSAs Primary glioblastoma tissue was also used for RNAseq to identify over --/highly expressed genes in the tumour, which were considered as TAAs PBMCs were used for DNA sequencing to identify the HLA genotype of the patient HLA binding was predicted for TSAΓΓAA associated peptides from somatic mutants, over/highly expressed molecules, and reported glioblastoma antigens using NetMHCpan 4.0 Server and IEDB Analysis Resource and for the patient's HLA class I and II molecules 78 natural peptides (N peptides), including N peptides with 9 amino acids for HLA class I molecules (N class I peptides) and N peptides with 11 - 13 amino acids for HLA class II molecules (N class II peptides), were selected based on predicted HLA binding affinities Designed mutations with the intent to increase the stimulatory strength of N peptides by single amino acid substitutions of putative peptide to TCR contact positions for TCR were introduced These are referred to as designed neopeptides (D neopeptides). A total of 175 peptides, including N peptides (n=78) and D neopeptides (n=97) were selected as the peptide library for preparing vaccine peptide cocktails. (B) Mutation strategy for designing D neopeptides for HLA class I molecules (D class I neopeptide). Using HLA A* 26 01 as an example, putative peptide anchor positions 2, 6 and 9 as well as preferred amino acids for HLA A* 26 01 are reported in the database of SYFPEITHI, which means that other positions, in particular positions 3 4 and 5 are potential peptide to TCR contact positions. Conservative or non-conservative amino acid substitutions were implemented in one of these positions for each N class I peptides, such as the N class I peptide I_RNA_ 14_0 and its corresponding D class I neopeptide I_RNA_ 14_1. (C) Mutation strategy for designing D neopeptides for HLA class II molecules. (D class II neopeptide). The strategy is similar to designing D class I neopeptides, however, before analysing the peptide anchor/contact positions for HLA/TCR, the 9 amino acid binding motifs of N class II peptides were analysed using NetMHCII 2:3 Server and/or SYFPHEITI

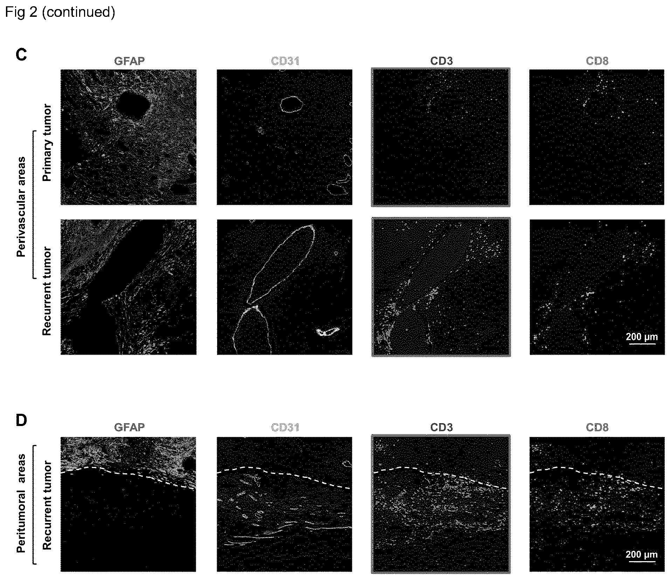

- Fig. 2: shows increased T lymphocyte infiltration in the recurrent compared to the primary tumour. (A-B) H&E and CD3 immunohistochemical staining in the primary (A) and recurrent tumour (B). The regions that are selected to show the CD3 immunohistochemical staining are indicated in the H&E stained tissue sections. (C-D) Expressions of GFAP (glioblastoma cells), CD31 (vascular epithelial cells), CD3 (T lymphocytes), and CD 8 (CD8⁺T cells) in the perivascular areas of primary and recurrent tumour C) and peritumoral areas of the recurrent tumour (D) were detected by IMC. Scale bars are indicated in the figures.

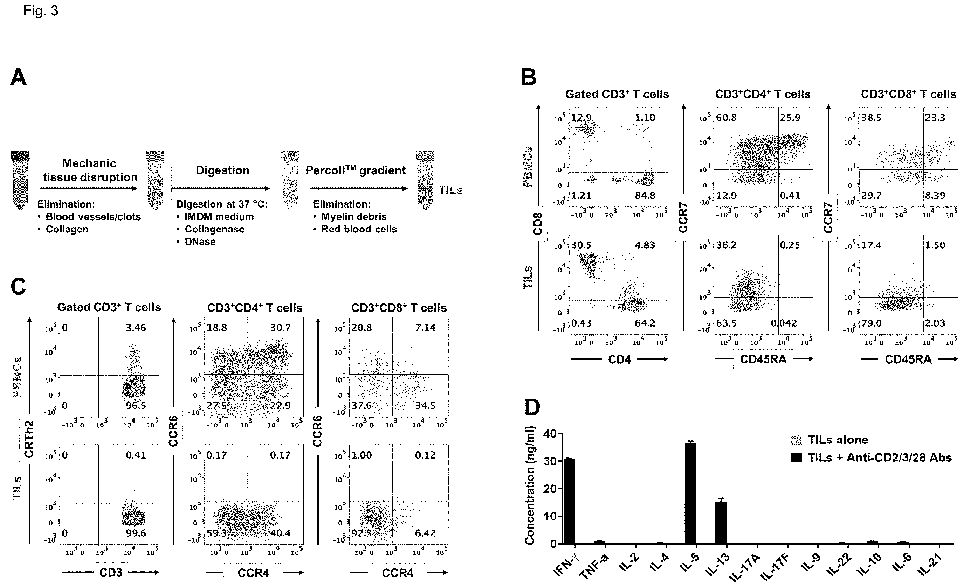

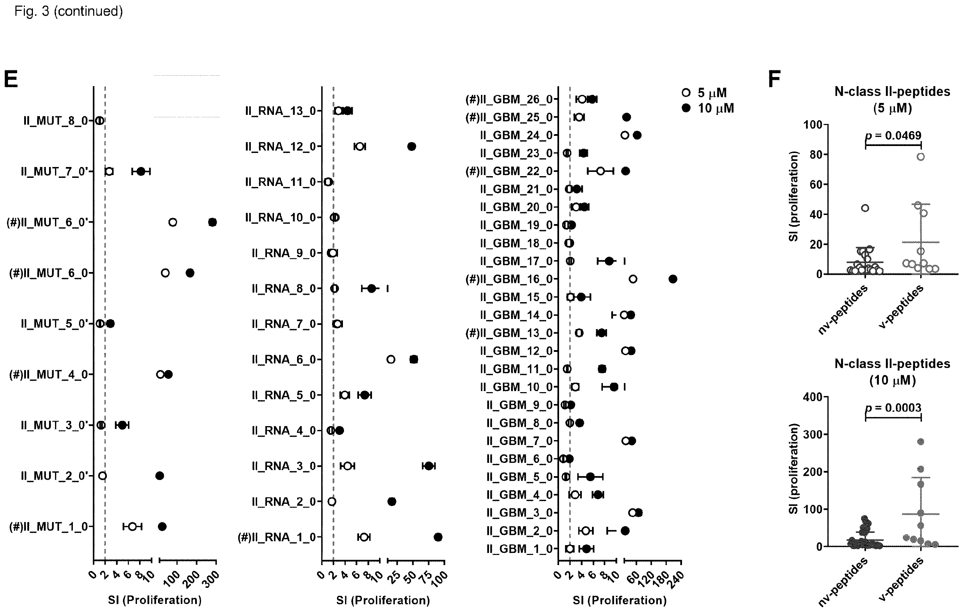

- Fig. 3: shows tumour infiltrating lymphocytes (TILs) display a Th1 /Th2 mixed phenotype and respond strongly to N class II peptides. (A) The procedure of isolating TILs from the recurrent glioblastoma (B) Phenotypes and frequencies of naïve and memory CD4⁺ and CD8⁺ T cell subsets in PBMCs and TILs, which were collected after the 4^{th} peptide cocktail vaccination, were detected by FACs (C) Functional phenotypes of naïve and memory CD4⁺ and CD8⁺ T cells in PBMCs and TILs were detected by FACs TILs primarily show a Th1 (CCR6⁻CCR4⁻CRTh2⁻CD4⁺)/Th2(CCR6⁻CCR4⁺CRTh2⁻CD4⁺)-mixed phenotype. (D) TILs were stimulated with anti CD2/CD3 /CD28 beads for 5 days, and cytokines in the supernatants measured with a bead-based immunoassay LEGENDplex multi analyte flow assay kit) (E) TILs were stimulated with 5 or 10 µM N-class II peptides for 5 days, and proliferation was measured by ³H-thymidine incorporation assay. The proliferation strength is depicted as stimulation index (SI). The red dotted line indicates a stimulatory response of SI=2 and values above the red dotted line were considered positive indicates the peptide that was used in the vaccinations (F) Comparison of the proliferation levels of TILs after stimulation with positive nv and v-N-class II peptides at the concentrations of 5 or 10 µM. (#) indicates the peptide that was used in the 1^{st} to 4^{th} vaccinations Data are expressed as mean ± SEM, and p values were determined using an unpaired t-test.

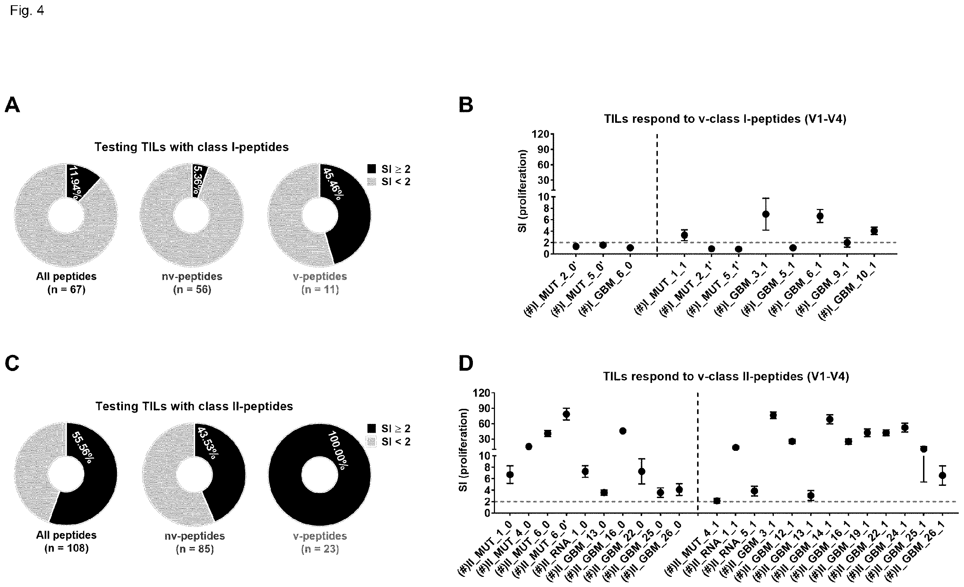

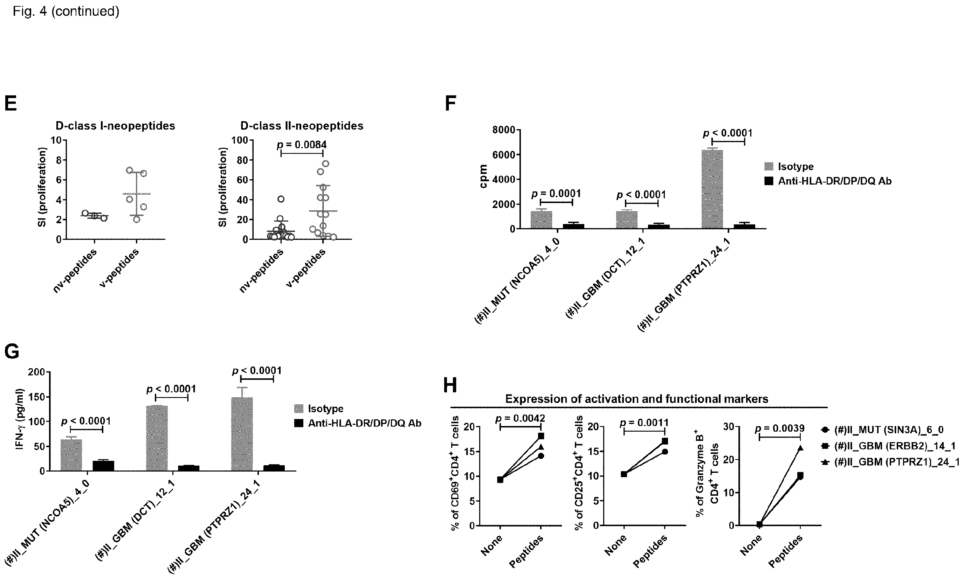

- Fig. 4: shows TILs preferentially respond to v-peptides. (A) Percentages of peptides among all predicted class I peptides, nv class I peptides, and v class I peptides that stimulated the TILs. (B) TILs were stimulated with 5 µM v class I peptides used in the 1^{st} to 4^{th} vaccinations for 5 days, and proliferation was measured by ³H-thymidine incorporation assay. (C) Percentages of peptides among all predicted class II peptides, nv class II peptides, and v class II peptides that stimulated the TILs. (D) TILs were stimulated with 55µM v class II peptides used in the 1^{st} to 4^{th} vaccinations for 5 days, and proliferation was measured by ³H-thymidine incorporation assay (E) Comparison of the proliferation levels of TILs after stimulation with positive nv and v D class I/II neopeptides (concentration 5 µM) (F-G) TILs were stimulated with v class II peptides in the presence of a blocking anti HLA DR/DP/DQ (TÜ 39 antibody or isotype control. Proliferations of TILs (F) and concentrations of IFN-*γ* in the supernatant (G) were detected 5 days after stimulation. (H) Expression levels of activation markers CD69 and CD25 as well as cytotoxicity marker Granzyme B on/in CD4⁺ TILs after stimulation with v class II peptides for 5 days. (#) indicates the peptide that was used in the 1st to 4th vaccinations Data are expressed as mean SEM, and p values were determined using an unpaired t test

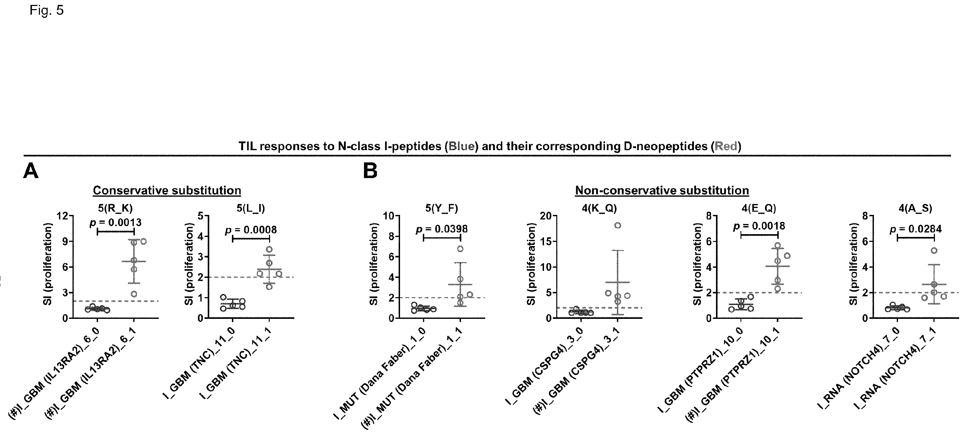

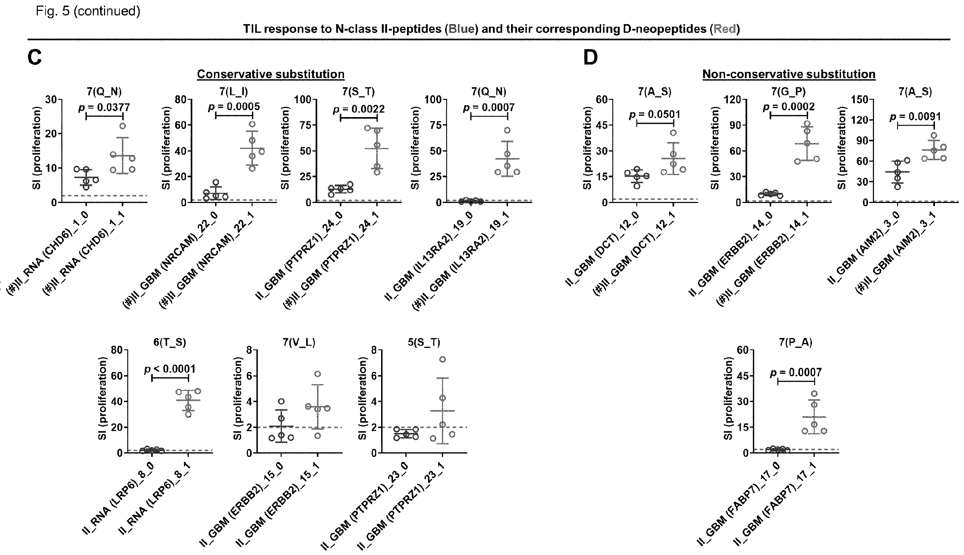

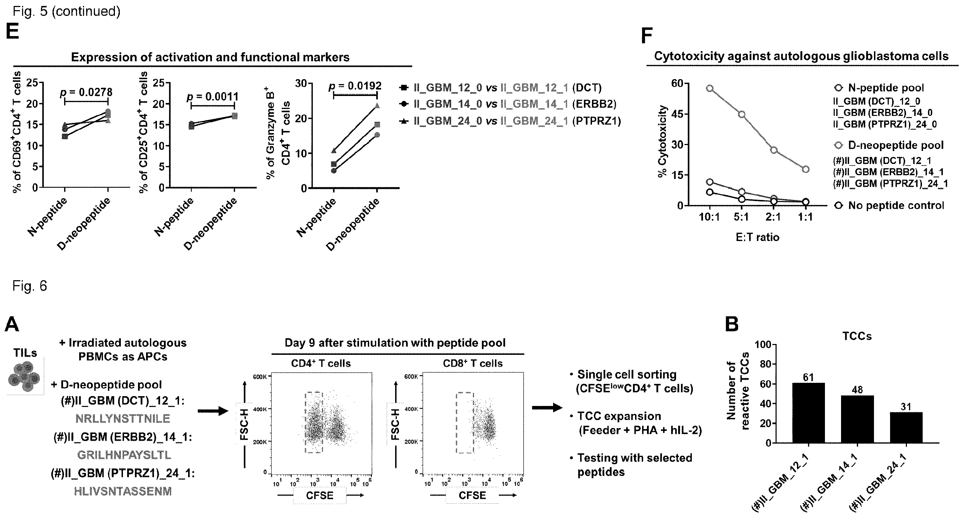

- Fig. 5: shows single amino acid substitutions increase the stimulatory effect of designed D neopeptides. (A-B) Comparison of the stimulatory strengths of N class I peptides and their corresponding D neopeptides by conservative (or non-conservative) amino acid substitution to TILs. (C-D) Comparison of the stimulatory strengths of N class II peptides and their corresponding D neopeptides by conservative (or non-conservative) amino acid substitution to TILs. (E) Comparison of the expression levels of activation markers CD69 and CD25 as well as cytotoxicity marker Granzyme B on/in CD4⁺ TILs after stimulation with N class II peptides and their corresponding D neopeptides. (F) Comparison of the cytotoxicity of TILs to tumour cells after stimulation with N class II peptide and D class II neopeptide pools for 5 days (indicates the peptide that was used in the 1^{st} to 4^{th} vaccinations Data are expressed as mean SEM, and p values were determined using an unpaired t test.

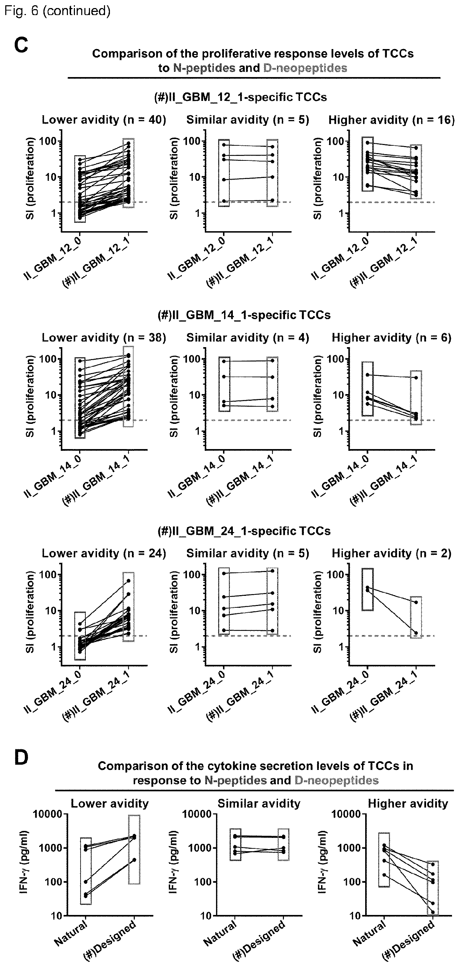

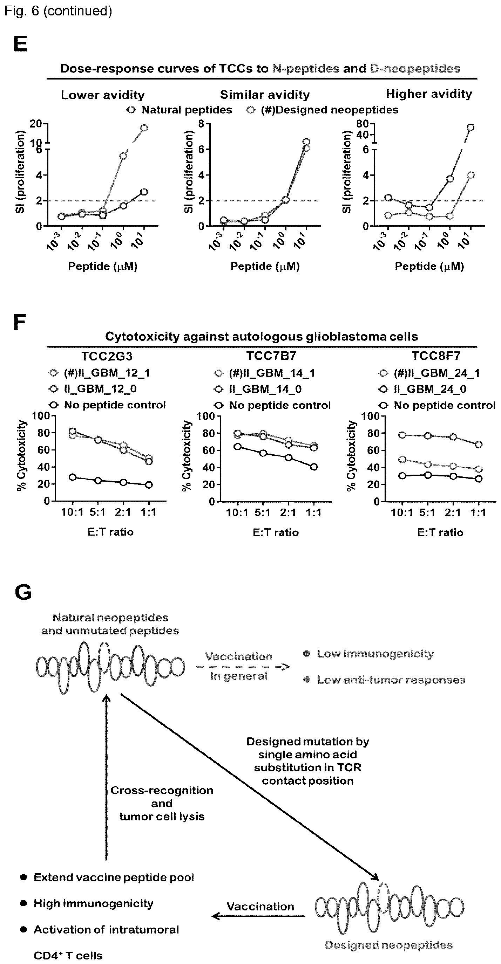

- Fig. 6: shows D neopeptide specific CD4⁺ T cells cross react with the corresponding N peptides. (A) Procedure of generating D class II neopeptide specific CD4⁺TCCs. CD4⁺ TCCs were isolated from TILs after co culture with irradiated autologous PBMCs as APCs and stimulation with pooled vaccine D class II neopeptides II_GBM_ 14_1 II_GBM_ 12_1 and II_GBM_ 24_1. (B) CD4⁺ TCCs were stimulated with 5µM of the indicated individual peptides for 3 days, and then proliferation was measured by 3 H thymidine incorporation assay Number of CD4⁺ TCCs responding to II_GBM_ 14 1 II_GBM_ 12 1 or !!_GBM_ 24_1. (C-E) CD4⁺ TCCs were stimulated with 5µM of the indicated individual peptides for 3 days, and then proliferation was measured by 3 H thymidine incorporation assay Three different patterns of cross reactivity between N peptides and D neopeptides for CD4⁺ TCCs: CD4⁺ TCCs with lower avidity for N peptides as compared to D neopeptides (lower avidity) CD4⁺ TCCs with similar avidity for N peptides and D neopeptides (similar avidity) and CD4⁺ TCCs with higher avidity for N peptides as compared to D neopeptides (higher avidity)(C). Concentrations of IFN γ in the supernatant were detected by ELISA(D). Dose response curves of CD4⁺ TCCs to N peptides and D neopeptides (E). (F) Comparison of the cytotoxicity of CD4⁺ TCCs to tumour cells after stimulation with N peptides or D neopeptides for 4 days. (G) Working model of how designed D neopeptides elicit cross reactive CD 4 T cells that also respond to the natural neopeptides and unmutated N peptides and mediate tumour cell lysis (indicates the peptide that was used in the 1^{st} to 4^{th} vaccinations

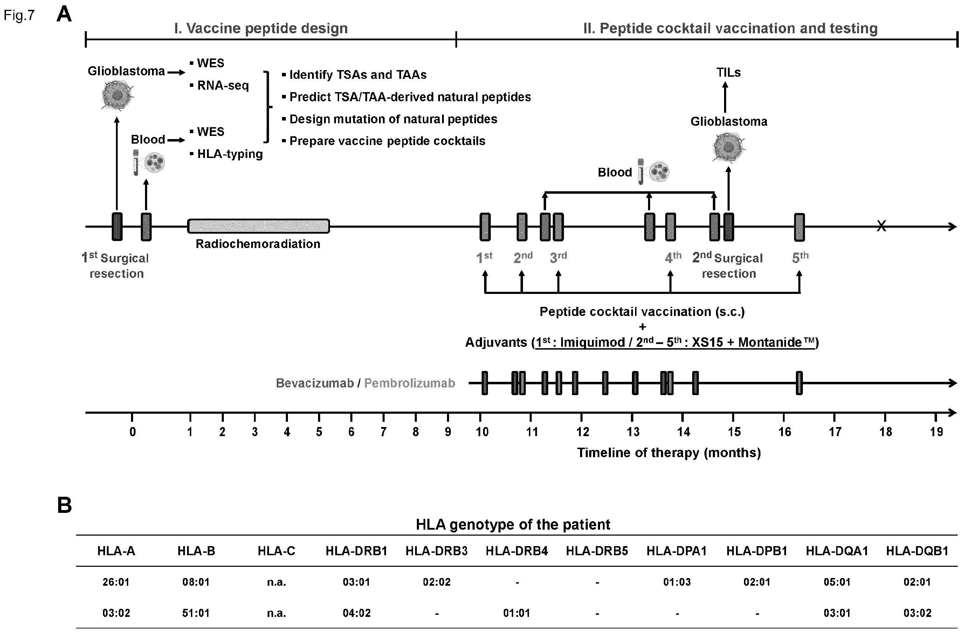

- Fig. 7: shows schematic outline of the personalized peptide vaccination. (A) Timeline of clinical events for a patient with glioblastoma in whom we applied a highly personalized peptide vaccination. I Vaccine peptide design Surgically resected primary glioblastoma and PBMCs were collected. Somatic mutants and over-/highly expressed genes in glioblastoma were identified as TSAs and TAAs by WES and RNAseq, and the HLA genotype were identified with PBMCs by DNA sequencing Potential vaccine peptides were predicted and selected from TSAs and TAAs based on HLA binding prediction Peptide cocktails containing 25 peptides for HLA class I or II molecules were prepared for vaccination. II Peptide cocktail vaccination and testing. The patient was s.c. vaccinated with the peptide cocktails, and Imiquimod or XS 15 Montanide TM were used as the adjuvant. Meanwhile, the patient received treatment with pembrolizumab and bevacizumab PBMCs were collected after vaccination at the indicated time points and tested with vaccine peptides to examine the effect of immunotherapy Surgically resected recurrent glioblastoma was collected after four vaccinations, and tumour infiltrating lymphocytes were isolated and tested with vaccine peptides (B) HLA genotypes of the patient with glioblastoma, including HLA-A, HLA-B, HLA-DR, HLA-DP, and HLA-DQ alleles, n.a. indicates not available

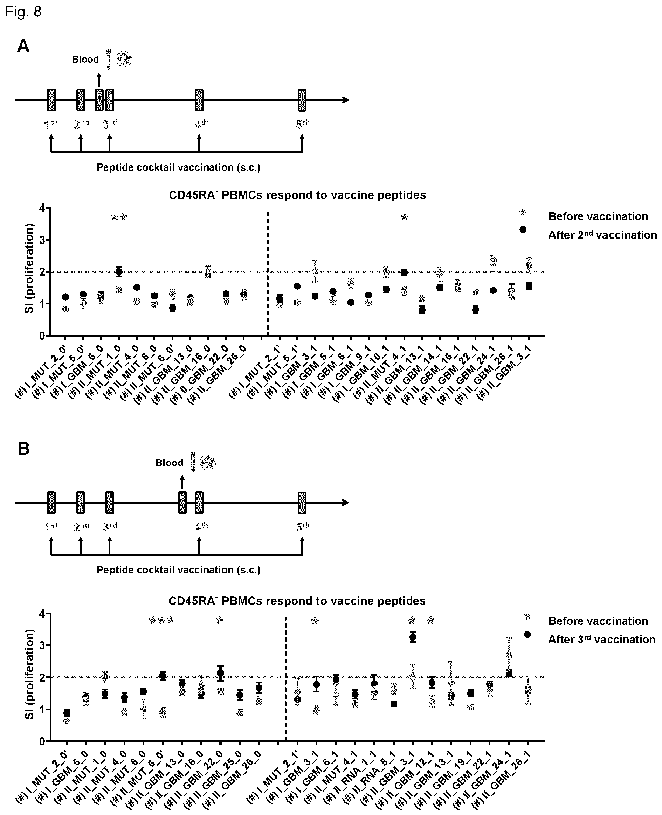

- Fig. 8: shows Peripheral blood T cell responses to v peptides increase with repetitive vaccination. (A) PBMCs were collected 3 weeks after the 2^{nd} peptide cocktail vaccination, and CD45RA-negative PBMCs were stimulated with 5µM v peptides for 7 days, and proliferation measured by ³H thymidine incorporation assay CD45RA negative PBMCs collected before vaccination were used as reference The proliferation strength is depicted as stimulation index (SI). The red dotted line indicates a stimulatory response of SI=2 and values above the red dotted line were considered positive indicates the peptide that was used in the vaccinations. (B) PBMCs were collected 5 weeks after the 3rd peptide cocktail vaccination, and CD45RA negative PBMCs were stimulated with 5µM v-peptides for 7 days, and proliferation was measured by ³H thymidine incorporation assay CD45RA negative PBMCs collected before vaccination were used as the control Data are expressed as mean SEM, and *p* values were determined using an unpaired t test **p*<0.05, ***p* <0.01, ****p*<0.001.

### Examples

### Example 1: Personalised cancer vaccination strategy

An altered peptide ligand (APL) of myelin basic protein (MBP), was previously developed by the inventors to block disease activity by partial agonist- or antagonist activity in multiple sclerosis (MS) patients, incorporated herein by reference (Bielekova B et. al. Nat Med 2000;6:1167-75). However, in a phase IIa trial, a strong increase of disease activity and inflammation was observed in the brain in 3/8 patients receiving the highest dose of the APL peptide. Mechanistic studies showed that APL-immunization activated CD4⁺ T cells that cross-recognized both the APL, the equivalent of a neopeptide, but also the natural unmutated MBP peptide. Interestingly, the responses of some T cell clones against the natural peptide were significantly higher than against the "foreign", artificial APL peptide. These findings were remarkable as MBP is expressed in the thymus and high avidity MBP-specific T cells undergo negative selection. Indeed, the MBP-specific T cells others have examined show only low avidity for the self-peptide (Li Y et al., EMBO J 2005;24:2968-79; Nicholson MJ et al., Immunity 2005;23:351-60).

Based on these findings, the inventors reasoned artificial peptides based on proteins expressed within tumours could be designed to stimulate both CD4⁺ and CD8⁺ T cell responses against multiple tumour targets. In the context of a vaccine to multiple cancer targets within an individual, these peptides should bind to autologous HLA class I and -II molecules, should be adaptable during the disease course based on immune reactivity, and should induce high avidity T cell responses. Towards this aim, tumour-specific antigens (TSAs) and tumour-associated antigens (TAAs) were first identified based on somatic mutations and over-/highly expressed genes of the autologous tumour respectively from a patient with glioblastoma and some previously reported glioblastoma antigens (Phuphanich S et al., Cancer Immunol Immunother 2013;62:125-35; Dutoit V. et al., Oncoimmunology 2018;7:e1391972; Dutoit V. et al., Brain 2012;135:1042-54). Peptides from TSAs and TAAs were then chosen based on predicted binding to the HLA class I and -II molecules of the patient. Most importantly, considering the low mutational load of glioblastoma and the low immunogenicity of self-proteins, neopeptides were created by targeted mutation of putative T cell receptor (TCR) contact amino acids (AAs) in the selected peptides. This approach has two aims: to increase the number of possible target antigens, and to increase immunogenicity rather than relying on the often poorly immunogenic and/or poorly HLA-binding peptides derived from somatic mutations of the tumour.

### Example 2: Prediction strategy and designed mutations of TSNT AA-derived peptides

A personalized vaccination was performed in a 55-year-old patient with glioblastoma **(****Fig. 7A****).** The cocktail of peptides was developed to efficiently stimulate novel CD4⁺ and CD8⁺ T cell responses **(****Fig. 1A****).** TSAs that included somatic mutants were identified using whole-exome sequencing (WES) of tumour tissue and peripheral blood mononuclear cells (PBMCs) and TAAs that were over-/highly expressed in the primary tumour using RNA sequencing (RNAseq). 40 somatic mutants were identified in the tumour. Further, over-/highly expressed genes were identified by comparison with glioblastomas in The Cancer Genome Atlas (TCGA) database. In addition, 12 TAAs reported in the context of glioblastoma based on immunopeptidome- or sequencing analyses were also included as a basis peptide prediction **(Table 1).**

**Table 1: Literature derived, glioblastoma associated TAAs**

| **Gene** | **mRNA level (TPM) in patient's primary tumour** | **Main references** |
|---|---|---|
| AIM2*** | 51.35 | *Oncoimmunology. 2018 Nov 7;7(2):e1391972. |
| BCAN* | 25292.51 | |
| CSPG4* | 1462.12 | |
| ERBB2* | 1417.51 | |
| FABP7** | 9162.04 | **Brain. 2012 Apr135(Pt 4):1042-54. |
| IL13RA2*** | 68.60 | |
| NLGN4X** | 951.67 | ***Cancer Immunol Immunother. 2013 Jan;62(1):125-35. |
| NRCAM** | 7062.29 | |
| PTPRZ1 * | 44508.42 | |
| TNC* | 5288.30 | ****Clin Cancer Res.2014 May 15;20(10):2684-94. |
| CMV_pp65*** * | / | |

Next, using NetMHCpan 4.0 Server and IEDB Analysis Resource, TSA/TAA-derived natural peptides (N-peptides) were identified with predicted high/moderate binding affinity and HLA-motifs for the autologous HLA class I and -II molecules **(****Fig. 1A****;** **Fig. 7B****).** 78 TSA/TAA-derived N-peptides, including peptides with 9 AAs for HLA class I molecules (N-class I-peptides) and peptides with 11-13 AAs for HLA class II molecules (N-class II-peptides) **(Table 2),** were selected from 10 somatic mutants, 22 over-/highly expressed genes **(Table 3),** and 12 reported glioblastoma antigens **(Table 1).** N-class I-peptides included: (i) 4 neopeptides that contain naturally mutated AA from TSAs (I_MUT_X_0'), (ii) one unmutated peptide from a TSA (I_MUT_X_0), (iii) 15 unmutated peptides from over-/highly expressed TAAs (I_RNA_X_0), and (iv) 11 unmutated peptides from reported glioblastoma antigens (I_GBM_X_0). N-class II-peptides included: (i) 5 neopeptides that contain naturally mutated AA from TSAs (II_MUT_X_0'), (ii) 3 unmutated peptides from TSAs (II_MUT_X_0), (iii) 13 unmutated peptides from over-/highly expressed TAAs (II_RNA_X_0), and (iv) 26 unmutated peptides from reported glioblastoma antigens (II_GBM_X_0) **(Table 2).** The peptide nomenclature is depicted in: I or II indicate if the peptides were chosen/designed for HLA class I or -II binding; MUT/RNA/GBM indicates a naturally mutated peptide (MUT), derivation from the over-/highly expressed genes (RNA) or from known glioblastoma (GBM) targets; X indicates the number of the peptide; 0 or 1 indicate if the peptides had been artificially mutated (1) or not (0); (') indicates that peptides contained the naturally mutated amino acid.

**Table 2: Sequences according to the ST25 sequences listing.**

| **TSA/TAA-derived N-peptides and D-neopeptides (Class-I-peptides)** | | | | | | | | | |
|---|---|---|---|---|---|---|---|---|---|
| **Peptide ID** | **Sequence** | **SE Q ID NO** | **Source protein** | **Potential binding HLA** | **V_ 1st** | **V_ 2nd** | **V_ 3rd** | **V_ 4th** | **V_ 5th** |
| I_GBM_1_0 | SPRVKWTFL | 001 | BCAN | HLA-B*08:01 | | | | | |
| I_GBM_1_1 | SPRLKWTFL | 002 | | HLA-B*08:01 | | | | | |
| I_GBM_2_0 | EMISVLGPI | 003 | CMV_pp 65 | HLA-A*26:01 | | | | | |
| I_GBM_2_1 | EMISLLGPI | 004 | | HLA-A*26:01 | | | | | |
| I_GBM_3_0 | DLRKRRVLF | 005 | CSPG4 | HLA-B*08:01 | | | | | |
| I_GBM_3_1 | DLRQRRVLF | 006 | | HLA-B*08:01 | # | # | # | # | √ |
| I_GBM_3_2 | DLRNRRVLF | 007 | | HLA-B*08:01 | | | | | |
| I_GBM_4_0 | CTIDVYMIM | 008 | ERBB2 | HLA-A*26:01 | | | | | |
| I_GBM_4_1 | CTIDLYMIM | 009 | | HLA-A*26:01 | | | | | |
| I_GBM_5_0 | MPNQAQMRI | 010 | | HLA-B*51:01 | | | | | |
| I_GBM_5_1 | MPQQAQMRI | 011 | | HLA-B*51:01 | # | # | | | |
| I_GBM_6_0 | NIGCRFPYL | 012 | IL13RA2 | HLA-B*08:01 | # | # | # | # | |
| I_GBM_6_1 | NIGCKFPYL | 013 | | HLA-B*08:01 | # | # | # | # | √ |
| I_GBM_7_0 | MPKKKRLSA | 014 | NRCAM | HLA-B*08:01 | | | | | |
| I_GBM_7_1 | MPKQKRLSA | 015 | | HLA-B*08:01 | | | | | |
| I_GBM_7_2 | MPKAKRLSA | 016 | | HLA-B*08:01 | | | | | |
| I_GBM_8_0 | VVVANVSKY | 017 | | HLA-A*26:01 | | | | | |
| I_GBM_8_1 | VVVAQVSKY | 018 | | HLA-A*26:01 | | | | | |
| I_GBM_9_0 | HTIKDFWRM | 019 | PTPRZ1 | HLA-A*26:01 | | | | | |
| I_GBM_9_1 | HTIKEFWRM | 020 | | HLA-A*26:01 | # | # | | | |
| I_GBM_10_0 | TMIEKFAVL | 021 | | HLA-B*08:01 | | | | | |
| I_GBM_10_1 | TMIQKFAVL | 022 | | HLA-B*08:01 | # | # | | # | |
| I_GBM_10_2 | TMIDKFAVL | 023 | | HLA-B*08:01 | | | | | |
| I_GBM_11_0 | TTIGLLYPF | 024 | TNC | HLA-A*26:01 | | | | | |
| I_GBM_11_1 | TTIGILYPF | 025 | | HLA-A*26:01 | | | | | |
| I_RNA_1_0 | LLRNRLQAL | 026 | APOC4 | HLA-B*08:01 | | | | | |
| I_RNA_1_1 | LLRQRLQAL | 027 | | HLA-B*08:01 | | | | | |
| I_RNA_2_0 | FLRYRPLIL | 028 | CNTFR | HLA-B*08:01 | | | | | |
| I_RNA_2_1 | FLRWRPLIL | 029 | | HLA-B*08:01 | | | | | |
| I_RNA_3_0 | FLRRKAEAM | 030 | CSF1R | HLA-B*08:01 | | | | | |
| I_RNA_3_1 | FLRKKAEAM | 031 | | HLA-B*08:01 | | | | | |
| I_RNA_4_0 | TVIEDMLAM | 032 | GAS1 | HLA-A*26:01 | | | | | |
| I_RNA_4_1 | TVIEEMLAM | 033 | | HLA-A*26:01 | | | | | |
| I_RNA_5_0 | MPRDILIVV | 034 | GATM | HLA-B*51:01 | | | | | |
| I_RNA_5_1 | MPKDILIVV | 035 | | HLA-B*51:01 | | | | | |
| I_RNA_6_0 | MPFRLKDPV | 036 | L3MBTL 3 | HLA-B*51:01 | | | | | |
| I_RNA_6_1 | MPYRLKDPV | 037 | | HLA-B*51:01 | | | | | |
| I_RNA_7_0 | FPRARTVSV | 038 | NOTCH4 | HLA-B*08:01 | | | | | |
| I_RNA_7_1 | FPRSRTVSV | 039 | | HLA-B*08:01 | | | | | |
| I_RNA_8_0 | MPISSVRFV | 040 | PBRM1 | HLA-B*51:01 | | | | | |
| I_RNA_8_1 | MPLSSVRFV | 041 | | HLA-B*51:01 | | | | | |
| I_RNA_9_0 | TSINNMAAF | 042 | PTCH2 | HLA-A*26:01 | | | | | |
| I_RNA_9_1 | TSINQMAAF | 043 | | HLA-A*26:01 | | | | | |
| I_RNA_10_0 | HTIEDFWRM | 044 | PTPRA | HLA-A*26:01 | | | | | |
| I_RNA_10_1 | HTIEEFWRM | 045 | | HLA-A*26:01 | | | | | |
| I_RNA_11_0 | MAFANSPWV | 046 | ROCK1 | HLA-B*51:01 | | | | | |
| I_RNA_11_1 | MAMANSPWV | 047 | | HLA-B*51:01 | | | | | |
| I_RNA_12_0 | MPYTSMPMM | 048 | SETBP1 | HLA-B*51:01 | | | | | |
| I_RNA_12_1 | MPFTSMPMM | 049 | | HLA-B*51:01 | | | | | |
| I_RNA_13_0 | EIIQPLLDM | 050 | SFRS15 | HLA-A*26:01 | | | | | |
| I_RNA_13_1 | EIIQPLIDM | 051 | | HLA-A*26:01 | | | | | |
| I_RNA_14_0 | DVISNIETF | 052 | SOX9 | HLA-A*26:01 | | | | | |
| I_RNA_14_1 | DVISQIETF | 053 | | HLA-A*26:01 | | | | | |
| I_RNA_15_0 | FMRKRIEAI | 054 | TGFB2 | HLA-B*08:01 | | | | | |
| I_RNA_15_1 | FMRQRIEAI | 055 | | HLA-B*08:01 | | | | | |
| I_MUT_1_0 | HAIHYVNKI | 056 | From Dana Farber | HLA-A*26:01 | | | | | |
| I_MUT_1_1 | HAIHFVNKI | 057 | | HLA-A*26:01 | | | | # | |
| I_MUT_2_0' | LPKGFLASL | 058 | NCOA5 | HLA-B*08:01 | # | # | # | | |
| I_MUT_2_1' | LPKPFLASL | 059 | | HLA-B*08:01 | # | # | # | # | |
| I_MUT_2_2' | LPKAFLASL | 060 | | HLA-B*08:01 | | | | | |
| I_MUT_3_0' | AARARLHPL | 061 | B4DX03 | HLA-B*08:01 | | | | | |
| I_MUT_3_1' | AARSRLHPL | 062 | | HLA-B*08:01 | | | | | |
| I_MUT_4_0' | LRRLHGTTL | 063 | | HLA-B*08:01 | | | | | |
| I_MUT_4_1' | LRRIHGTTL | 064 | | HLA-B*08:01 | | | | | |
| I_MUT_5_0' | IMRGRGRGV | 065 | RBM33 | HLA-B*08:01 | # | # | | | |
| I_MUT_5_1' | IMRPRGRGV | 066 | | HLA-B*08:01 | # | # | | | |
| I_MUT_5_2' | IMRARGRGV | 067 | | HLA-B*08:01 | | | | | |

| **Predicted TSA/TAA-derived N-peptides and D-neopeptides (Class-II-peptides)** | | | | | | | | | |
|---|---|---|---|---|---|---|---|---|---|
| **Peptide ID** | **Sequence** | **SE Q ID NO** | **Source protein** | **Potential binding HLA** | **V_ 1st** | **V_ 2nd** | **V_ 3rd** | **V_ 4th** | **V_ 5th** |
| II_GBM_1_0 | KFFLSDEFNIAT | 068 | AIM2 | DPA1*01:03/DPB1*0 2:01 | | | | | |
| II_GBM_1_1 | KFFLSEEFNIAT | 069 | | DPA1*01:03/DPB1*0 2:01 | | | | | |
| II_GBM_2_0 | AAIRNDVAKQR AA | 070 | | DRB1*03:01 | | | | | |
| II_GBM_2_1 | AAIRNDLAKQR AA | 071 | | DRB1*03:01 | | | | | |
| II_GBM_3_0 | TLMIQNAGAVS AV | 072 | | DRB1*04:02 | | | | | √ |
| II_GBM_3_1 | TLMIQNSGAVS AV | 073 | | DRB1*04:02 | # | # | # | # | √ |
| II_GBM_4_0 | LRYEVDTVLRY EV | 074 | BCAN | DRB1*03:01 | | | | | |
| II_GBM_4_1 | LRYEVDSVLRY EV | 075 | | DRB1*03:01 | | | | | |
| II_GBM_5_0 | FFFDIDLLLQR | 076 | CMV_pp 65 | DPA1*01:03/DPB1*0 2:01 | | | | | |
| II_GBM_5_1 | FFFDIELLLQR | 077 | | DPA1*01:03/DPB1*0 2:01 | | | | | |
| II_GBM_6_0 | NDIYRIFAELE | 078 | | DQA1*05:01/DQB1*0 2:01 | | | | | |
| II_GBM_6_1 | NDIYRIYAELE | 079 | | DQA1*05:01/DQB1*0 2:01 | | | | | |
| II_GBM_6_2 | NDIYKIFAELE | 080 | | DQA1*05:01/DQB1*0 2:01 | | | | | |
| II_GBM_7_0 | LKMLNIPSINVH H | 081 | | DRB1*04:02 | | | | | √ |
| II_GBM_7_1 | LKMLHIPSINVH H | 082 | | DRB1*04:02 | | | | | √ |
| II_GBM_7_2 | LKMLQIPSINVH H | 083 | | DRB1*04:02 | | | | | |
| II_GBM_8_0 | SIYVYALPLKML N | 084 | | DRB3*02:02 | | | | | |
| II_GBM_8_1 | SIYVYAVPLKM LN | 085 | | DRB3*02:02 | | | | | |
| II_GBM_9_0 | APLEGVLVELE VL | 086 | CSPG4 | DQA1*05:01/DQB1*0 2:01 | | | | | |
| II_GBM_9_1 | APLEGVIVELEV L | 087 | | DQA1*05:01/DQB1*0 2:01 | | | | | |
| II_GBM_9_2 | APLEAVLVELE VL | 088 | | DQA1*05:01/DQB1*0 2:01 | | | | | |
| II_GBM_10_ 0 | GSLTLAPPLLR VS | 089 | | DRB3*02:02 | | | | | |
| II_GBM_10_ 1 | GSLTLTPPLLR VS | 090 | | DRB3*02:02 | | | | | |
| II_GBM_11_ 0 | VLLAFLQYRRL R | 091 | DCT | DPA1*01:03/DPB1*0 2:01 | | | | | |
| II_GBM_11_ 1 | VLLTFLQYRRL R | 092 | | DPA1*01:03/DPB1*0 2:01 | | | | | |
| II_GBM_12_ 0 | NRLLYNATTNIL E | 093 | | DRB1*04:02 | | | | | √ |
| II_GBM_12_ 1 | NRLLYNSTTNIL E | 094 | | DRB1*04:02 | | | # | # | √ |
| II_GBM_13_ 0 | FGSLAFLPESF DG | 095 | ERBB2 | DQA1*05:01/DQB1*0 2:01 | # | # | # | # | |
| II_GBM_13_ 1 | FGSLAFIPESFD G | 096 | | DQA1*05:01/DQB1*0 2:01 | # | # | # | # | |
| II_GBM_13_ 2 | FGSLSFLPESF DG | 097 | | DQA1*05:01/DQB1*0 2:01 | | | | | |
| II_GBM_14_ 0 | GRILHNGAYSL TL | 098 | | DRB1*04:02 | | | | | |
| II_GBM_14_ 1 | GRILHNPAYSL TL | 099 | | DRB1*04:02 | # | # | | # | √ |
| II_GBM_14_ 2 | GRILHNAAYSL TL | 100 | | DRB1*04:02 | | | | | √ |
| II_GBM_15_ 0 | MSYLEDVRLVH RD | 101 | | DRB1*03:01 | | | | | |
| II_GBM_15_ 1 | MSYLEDLRLVH RD | 102 | | DRB1*03:01 | | | | | |
| II_GBM_16_ 0 | RLVHRDLAARN VL | 103 | | DRB1*03:01 | # | # | # | # | √ |
| II_GBM_16_ 1 | RLVHRDIAARN VL | 104 | | DRB1*03:01 | # | # | | # | √ |
| II_GBM_17_ 0 | LPFFLLPSFFN NT | 105 | FABP7 | DPA1*01:03/DPB1*0 2:01 | | | | | |
| II_GBM_17_ 1 | LPFFLLASFFN NT | 106 | | DPA1*01:03/DPB1*0 2:01 | | | | | |
| II_GBM_18_ 0 | PSFFNNTSLAR FF | 107 | | DRB3*02:02 | | | | | |
| II_GBM_18_ 1 | PSFFNNSSLAR FF | 108 | | DRB3*02:02 | | | | | |
| II_GBM_19_ 0 | SSYFTFQLQNI V | 109 | IL13RA2 | DPA1*01:03/DPB1*0 2:01 | | | | | |
| II_GBM_19_ 1 | SSYFTFNLQNI V | 110 | | DPA1*01:03/DPB1*0 2:01 | | | # | # | √ |
| II_GBM_20_ 0 | TKFIHTKPNRF EE | 111 | NLGN4X | DRB3*02:02 | | | | | |
| II_GBM_20_ 1 | TKFIHTRPNRF EE | 112 | | DRB3*02:02 | | | | | |
| II_GBM_21_ 0 | VCVMLNSNVLL WL | 113 | | DRB1*04:02 | | | | | |
| II_GBM_21_ 1 | VCVMLNTNVLL WL | 114 | | DRB1*04:02 | | | | | |
| II_GBM_22_ 0 | NAFVNVLAEPP RI | 115 | NRCAM | DQA1*05:01/DQB1*0 2:01 | # | # | # | # | |
| II_GBM_22_ 1 | NAFVNVIAEPP RI | 116 | | DQA1*05:01/DQB1*0 2:01 | # | # | # | # | √ |
| II_GBM_22_ 2 | NAFVQVLAEPP RI | 117 | | DQA1*05:01/DQB1*0 2:01 | | | | | |
| II_GBM_23_ 0 | ASFSSELFRHL HT | 118 | PTPRZ1 | DPA1*01:03/DPB1*0 2:01 | | | | | |
| II_GBM_23_ 1 | ASFSTELFRHL HT | 119 | | DPA1*01:03/DPB1*0 2:01 | | | | | |
| II_GBM_24_ 0 | HLIVSNSASSE NM | 120 | | DRB1*04:02 | | | | | √ |
| II_GBM_24_ 1 | HLIVSNTASSE NM | 121 | | DRB1*04:02 | # | # | # | # | √ |
| II_GBM_25_ 0 | KHIRSQRNYLV QT | 122 | | DRB3*02:02 | | | | # | √ |
| II_GBM_25_ 1 | KHIRSQKNYLV QT | 123 | | DRB3*02:02 | | | # | # | √ |
| II_GBM_26_ 0 | RAEIVTEAEPE VD | 124 | TNC | DQA1*05:01/DQB1*0 2:01 | # | # | # | # | |
| II_GBM_26_ 1 | RAEIVTDAEPE VD | 125 | | DQA1*05:01/DQB1*0 2:01 | # | # | # | # | |
| II_GBM_26_ 2 | RAEIVSEAEPE VD | 126 | | DQA1*05:01/DQB1*0 2:01 | | | | | |
| II_RNA_1_0 | KTFDWTQFRIIS R | 127 | CHD6 | DPA1*01:03/DPB1*0 2:01 | | | | # | √ |
| II_RNA_1_1 | KTFDWTNFRIIS R | 128 | | DPA1*01:03/DPB1*0 2:01 | | | # | # | √ |
| II_RNA_2_0 | NKFLSEIEDELF N | 129 | CHD7 | DQA1*05:01/DQB1*0 2:01 | | | | | |
| II_RNA_2_1 | NKFLAEIEDELF N | 130 | | DQA1*05:01/DQB1*0 2:01 | | | | | |
| II_RNA_3_0 | VAAFAYQIEEL MI | 131 | CNTF | DQA1*05:01/DQB1*0 2:01 | | | | | √ |
| II_RNA_3_1 | VAAFAYNIEEL MI | 132 | | DQA1*05:01/DQB1*0 2:01 | | | | | |
| II_RNA_3_2 | VAAFSYQIEEL MI | 133 | | DQA1*05:01/DQB1*0 2:01 | | | | | |
| II_RNA_4_0 | LFFEFYRLLHD A | 134 | DNMT3A | DPA1*01:03/DPB1*0 2:01 | | | | | |
| II_RNA_4_1 | LFFDFYRLLHD A | 135 | | DPA1*01:03/DPB1*0 2:01 | | | | | |
| II_RNA_5_0 | FKIQYLQFLAY | 136 | DOT1L | DPA1*01:03/DPB1*0 2:01 | | | | | |
| II_RNA_5_1 | FKIQYLNFLAY | 137 | | DPA1*01:03/DPB1*0 2:01 | | | # | | |
| II_RNA_6_0 | REFTKATNVRL RF | 138 | LAMA5 | DRB3*02:02 | | | | | √ |
| II_RNA_6_1 | REFTKASNVRL RF | 139 | | DRB3*02:02 | | | | | √ |
| II_RNA_7_0 | GSFLRIPSLHLL L | 140 | LGI4 | DRB3*02:02 | | | | | |
| II_RNA_7_1 | GSFLKIPSLHLL L | 141 | | DRB3*02:02 | | | | | |
| II_RNA_8_0 | HPFALTLFEDIL Y | 142 | LRP6 | DPA1*01:03/DPB1*0 2:01 | | | | | |
| II_RNA_8_1 | HPFALSLFEDIL Y | 143 | | DPA1*01:03/DPB1*0 2:01 | | | | | √ |
| II_RNA_9_0 | LEFAQFPFLLR | 144 | PTCH2 | DPA1*01:03/DPB1*0 2:01 | | | | | |
| II_RNA_9_1 | LEFANFPFLLR | 145 | | DPA1*01:03/DPB1*0 2:01 | | | | | |
| II_RNA_10_ 0 | DVFQTVKSLRL QR | 146 | PTPRA | DRB3*02:02 | | | | | |
| II_RNA_10_ 1 | DVFQSVKSLRL QR | 147 | | DRB3*02:02 | | | | | |
| II_RNA_11_ 0 | LQFLADLEELIT | 148 | SETBP1 | DQA1*05:01/DQB1*0 2:01 | | | | | |
| II_RNA_11_ 1 | LQFLSDLEELIT | 149 | | DQA1*05:01/DQB1*0 2:01 | | | | | |
| II_RNA_12_ 0 | LQFLADLEELIT K | 150 | | DQA1*05:01/DQB1*0 2:01 | | | | | |
| II_RNA_12_ 1 | LQFLSDLEELIT K | 151 | | DQA1*05:01/DQB1*0 2:01 | | | | | |
| II_RNA_13_ 0 | MGFIQAMSALR GM | 152 | SFRS17 A | DRB3*02:02 | | | | | |
| II_RNA_13_ 1 | MGFIQSMSALR GM | 153 | | DRB3*02:02 | | | | | |
| II_MUT_1_0 | PALEAHCVEFL | 154 | BTBD2 | DQA1*05:01/DQB1*0 2:01 | # | # | # | # | |
| II_MUT_1_1 | PALEAHSVEFL | 155 | | DQA1*05:01/DQB1*0 2:01 | | | | | |
| II_MUT_2_0' | WLVLGFLWPLL TL | 156 | C5AR1 | DQA1*05:01/DQB1*0 2:01 | | | | | |
| II_MUT_2_1' | WLVLPFLWPLL TL | 157 | | DQA1*05:01/DQB1*0 2:01 | | | | | |
| II_MUT_3_0 | FRLKINEETPLK | 158 | GANC | DRB1*04:02 | | | | | |
| II_MUT_3_0' | FRLKINEGTPLK | 159 | | DRB1*04:02 | | | | | |
| II_MUT_3_1' | FRLKINQGTPL K | 160 | | DRB1*04:02 | | | | | |
| II_MUT_3_2' | FRLKINDGTPL K | 161 | | DRB1*04:02 | | | | | |
| II_MUT_4_0 | TGINFDNPSVQ KA | 162 | NCOA5 | DRB1*03:01 | # | # | # | # | √ |
| II_MUT_4_1 | TGINFDEPSVQ KA | 163 | | DRB1*03:01 | # | # | # | | |
| II_MUT_4_2 | TGINFDQPSVQ KA | 164 | | DRB1*03:01 | | | | | |
| II_MUT_5_0' | LQFVKDHPLMD DL | 165 | SEMA4D | DRB1*03:01 | | | | | |
| II_MUT_5_1' | LQFVKDQPLM DDL | 166 | | DRB1*03:01 | | | | | |
| II_MUT_5_2' | LQFVKDKPLMD DL | 167 | | DRB1*03:01 | | | | | |
| II_MUT_6_0 | QPVEFNHAINY VN | 168 | SIN3A | DRB1*04:02 | # | # | # | # | √ |
| II_MUT_6_0' | QPVEFNHAIHY VN | 169 | | DRB1*04:02 | # | # | # | # | √ |
| II_MUT_6_1 | QPVEFNQAINY VN | 170 | | DRB1*04:02 | | | | | |
| II_MUT_6_2 | QPVEFNKAINY VN | 171 | | DRB1*04:02 | | | | | |
| II_MUT_7_0' | RQLQVDRTKLS RA | 172 | STC2 | DRB1*03:01 | | | | | |
| II_MUT_7_1' | RQLQVDKTKLS RA | 173 | | DRB1*03:01 | | | | | |
| II_MUT_8_0 | PILSADDLLGSP | 174 | ZFP36L1 | DRB1*03:01 | | | | | |
| II_MUT_8_1 | PILSADELLGSP | 175 | | DRB1*03:01 | | | | | |

**Table 3: Over --/highly expressed genes that were chosen for the design of vaccine peptides**

| **Gene** | **mRNA level (TPM) in patient's primary tumour** | **Compared to glioblastoma cohort in TCGA Database** |
|---|---|---|
| APOC4 | 27.36 | Over-expression |
| CHD6 | 1481.9 | High-expression |
| CHD7 | 1596.8 | High-expression |
| CNTF | 70.29 | Over-expression |
| DNMT3A | 628.37 | High-expression |
| DOT1L | 1914.56 | High-expression |
| GAS1 | 2026.52 | High-expression |
| GATM | 8878.37 | High-expression |
| L3MBTL3 | 231.48 | High-expression |
| LAMA5 | 2663.72 | High-expression |
| LGI4 | 832.49 | High-expression |
| LRP6 | 1539.56 | High-expression |
| NOTCH4 | 1238.22 | High-expression |
| PBRM1 | 1200.76 | High-expression |
| PTCH2 | 427.19 | Over-expression |
| PTPRA | 7569.87 | Over-expression |
| ROCK1 | 1452.3 | High-expression |
| SETBP1 | 979.8 | High-expression |
| SFRS15 | 1183.5 | High-expression |
| SFRS17A | 1289.98 | High-expression |
| SOX9 | 9164.98 | High-expression |
| TGFB2 | 3541.25 | High-expression |

As a key step of this study, mutations were introduced in critical AA positions of N-peptides to increase the number of neopeptides available for vaccination and to enhance immunogenicity. Designed mutations were introduced by first positioning peptides in the peptide-binding grooves of HLA class I and II molecules using known binding motifs in the database of SYFPEITHI and then introducing single conservative, neutral, or non-conservative AA exchanges in putative TCR contact positions **(**Fig. 1B and C). These artificially mutated peptides are referred to as designed neopeptides (D-neopeptides). The nomenclature of the D-neopeptides is as follows: I/II_MUT/RNA/GBM_X_1/2 **(Table 2).** Suffixes 1 or 2 are used to distinguish between the up to two D-neopeptides of the respective N-peptide. Overall, 175 peptides from the above sources were synthesized **(Table 2).**

### Example 3: Vaccination strategy

The schedule of the vaccination after standard surgical resection and radiochemotherapy as well as parallel treatment with pembrolizumab (anti-PD1 antibody) and bevacizumab (anti-VEGF-A antibody) is schematically depicted **(****Fig. 7A****).** Peptide cocktails containing ~25 TSA/TAA-derived peptides, including N-peptides and D-neopeptides for both HLA class I and II molecules **(Table 2),** were s.c. injected for vaccination, and the adjuvants Imiquimod (only first vaccination) or XS15 + Montanide^{™} were used to augment peptide-specific immune responses. No dexamethasone was given throughout the periods of vaccination.

### Example 4: Peripheral blood T cell responses to vaccine peptides increase with repetitive vaccination

PBMCs collected before vaccination were tested with all N-peptides (n = 78) and D-neopeptides (n = 97), however they exhibited little response to N-class I-peptides and N-class II-peptides, and D-neopeptides. Therefore, 25 peptides were chosen from somatic mutants or reported glioblastoma antigens for the 1^{st} and 2^{nd} vaccinations, and the ratio of N-peptide and D-neopeptide is around 1:2 **(Table 2).** In order to optimize the vaccine cocktail to induce strong immune responses, PBMC responses assessed the stimulatory capacity of the vaccine peptides (v-peptides) after vaccination. CD45RA-depleted (CD45RA⁻) PBMCs were the source of memory T cells. Cells collected 20 days after the 2^{nd} vaccination did not show stronger responses to v-peptides than CD45RA⁻ PBMCs collected before vaccination. Some responses were even lower **(****Fig. 8A****),** and were replaced in the 3^{rd} vaccination cocktail **(Table 2).** CD45RA⁻ PBMCs collected 40 days after the 3^{rd} vaccination showed increased responses to some v-peptides compared to CD45RA⁻ PBMCs collected before vaccination or after the 2^{nd} vaccination **(****Fig. 8B****).** However, PBMC responses to v-peptides remained overall modest **(****Fig. 8****),** which might be due to the low precursor frequency of v-peptide-specific T cells in blood, migration of vaccine-activated T cells into the tumour, or technical aspects, i.e., low sensitivity of the 3H-thymidine proliferation assay.

### Example 5: Increased T cell infiltration in perivascular areas of the recurrent tumour after peptide vaccination

One month after the 4^{th} vaccination, a recurrent tumour was resected, which allowed the inventors to examine the potential effects of peptide vaccination on tumour-infiltrating lymphocytes (TILs). TILs were studied in the primary and recurrent tumour by H&E staining, CD3 immunohistochemical staining, and imaging mass cytometry (IMC) for multi-marker analysis. Few CD3⁺ TILs were found in the parenchymal areas of both primary and recurrent tumour **(****Fig. 2A****_i and 2B_i).** However, compared with the primary tumour **(****Fig. 2A****_ii and 2C_top),** the recurrent tumour showed increased CD3⁺ TILs in the perivascular areas **(****Fig. 2B****_ii and 2C_bottom)** and peritumoral tissue **(****Fig. 2B****_iii and 2D).** Further, few CD8⁺ T cells were seen in the perivascular areas of the primary tumour **(****Fig. 2C****_top),** while the number of infiltrating CD8⁺ T cells increased in the recurrent tumour **(****Fig. 2C****_bottom).** In particular, CD4⁺ T cells (CD3⁺CD8⁻ T cells) were relatively abundant and infiltrated both perivascular and peritumoral areas of the recurrent tumour but not the primary tumour **(**Fig. 2C and D). These results indicate that v-peptides stimulated and expanded TILs after vaccination.

### Example 6: Composition, phenotype, and response to vaccine peptides of TILs

Next, TILs from the recurrent tumour were isolated **(****Fig. 3A****)** and characterized by immunophenotyping. Two-thirds of TILs were CD4⁺, one-third CD8⁺ T cells, and all CD45RA⁻ memory T cells **(****Fig. 3B****).** CD4⁺ T cells mainly showed a Th1(CRTh2⁻CCR6⁻CCR4⁻)/Th2(CRTh2⁻CCR6⁻CCR4⁺)-multifunctional phenotype, and CD8⁺ T cells were mainly Tc1(CRTh2⁻CCR6⁻CCR4⁻) cells **(****Fig. 3C****).** Accordingly, TILs released interferon (IFN)-γ, IL-5, and IL-13 after stimulation **(****Fig. 3D****).** TILs did not respond to N-class I-peptides in proliferation assays but reacted strongly and in a dose-dependent manner to most of the N-class II-peptides, including both v-peptides (labelled with #) and non-vaccine (nv)-peptides **(****Fig. 3E****).** However, compared to nv-peptides, TIL responses to v-peptides were much stronger **(****Fig. 3F****).** These results indicate that repetitive vaccination with a complex peptide cocktail containing D-neopeptides led to infiltration of proinflammatory CD4⁺ and CD8⁺ T cells in the tumour.

### Example 7: Peptide vaccination induces robust intratumoural CD4+ T cell responses

To address the above point in more detail, the response of the TILs to v-peptides used in the 1^{st} to 4^{th} vaccination rounds were compared, including both N-peptides and D-neopeptides, with nv-peptides for HLA class I and -II molecules. For class I-peptides, the response rate to nv-peptides was low (5.36%), and an almost 9 times better rate was observed to v-peptides (45.46%) **(****Fig. 4A****),** although the overall response was still low **(****Fig. 4B****).** For class II-peptides, TILs responded to 43.53% of the nv-peptides, and 100% of the v-peptides elicited strong responses **(**Fig. 4C and D). Importantly, v-D-class II-neopeptides stimulated much higher TIL responses compared to nv-D-class II-neopeptides **(****Fig. 4E****),** which also held for v-N-class II-peptides **(****Fig. 3F****).** Hence, TILs preferentially respond to v-peptides and more strongly to class II-peptides. To confirm that peptides stimulated the TILs in an HLA-dependent manner, blocking studies were performed with the anti-HLA-DR/DP/DQ antibody (TÜ39), which inhibited both proliferation and IFN-γ secretion of TILs after stimulation with v-class II-peptides **(**Fig. 4F and G) and confirmed HLA class II restriction of the TIL responses. Furthermore, activation with v-class II-peptides not only led to upregulation of activation markers, but also significantly increased the fraction of cytotoxic granzyme B⁺ CD4⁺ T cells **(****Fig. 4H****).**

### Example 8: D-neopeptides stimulate stronger TIL responses than N-peptides

The purpose of artificially designing D-neopeptides was to induce more robust T cell responses against N-peptides to foster T cell infiltration and lysis of tumour cells. Comparing the stimulatory strength of N-peptides with the corresponding D-neopeptides for TILs disclosed that the latter elicited stronger responses in most cases. For class I-peptides, some v-D-neopeptides, such as I_GBM_6_1 **(****Fig. 5A****),** I_MUT_1_1, I_GBM_3_1, and I_GBM_10_1 **(****Fig. 5B****),** induced positive TIL responses, but TILs did not respond to any of the corresponding N-peptides, indicating that vaccination with D-class I-neopeptides did not activate CD8⁺ T cells sufficiently against N-class I-peptides, and/or the precursor frequencies were too low for detection. This contrasted strongly to local TIL responses to N-class II-peptides and D-class II-neopeptides **(**Fig. 5C and D). D-class II-neopeptides elicited robust TIL responses, which applied to both peptides with conservative- **(****Fig. 5C****),** i.e., AA exchanges with similar physicochemical properties, including charge, hydrophobicity and size, but also neutral- or non-conservative substitutions **(****Fig. 5D****).** CD4⁺ TILs responded better to D-class I I-neopeptides compared to N-class II-peptides based on upregulation of activation markers **(****Fig. 5E****)** and cytotoxicity against autologous tumour cells **(****Fig. 5F****).** The overall stronger TIL responses to D-neopeptides lends support to the hypothesis that mutations in critical AA positions stimulate T cells that have escaped negative selection and are recognized with higher avidity than natural self-peptides.

Eliciting cross-reactive T cells against both D-neopeptides and N-peptides is a key aim of the vaccination approach. If T cells responding to D-neopeptides did not cross-react with the N-peptides that the tumour expresses, they would likely not be useful for the tumour defence. On the other hand, if D-neopeptides induced cross-reactive T cell responses against the N-peptides, especially against the unmutated N-peptides, it would open the possibility to target many more antigens in the tumour than through vaccination with a limited number of naturally occurring mutations, which may not be highly expressed in the tumour. Following this idea, the peptide groups in which D-neopeptides were used for vaccination but the corresponding N-peptides were not were analysed, including II_GBM_24_0 & II_GBM_24_1 **(****Fig. 5C****),** II_GBM_12_0 & II_GBM_12_1, II_GBM_14_0 & II_GBM_14_1, and II_GBM_3_0 & II GBM 3_1 **(****Fig. 5D****).** In these peptide groups, TIL responses to both N-peptides and D-neopeptides were positive. For some peptide pairs, including !!_RNA_8_0 & II_RNA_8_1, II_GBM_15_0 & II_GBM_15_1, II_GBM_23_0 & II GBM 23_1 **(****Fig. 5C****),** and II_GBM_17_0 & II_GBM_17_1 **(****Fig. 5D****),** when the D-neopeptide stimulated a positive TIL response but was not used for vaccination, TILs had no or a low response to the N-peptides. These data indicate that D-class II-neopeptide vaccination activates CD4⁺ T cells which are cross-reactive to their unmutated counterpart.

### Example 9; Vaccination with D-neopeptides induces cross-reactive T cell responses against N-peptides

Cross-reactivity against D-neopeptides and N-peptides cannot be measured at the level of bulk TILs, so T cell clones (TCCs) were generated using a pool of three exemplary D-class II-neopeptides, including II_GBM_12_1 from the dopachrome tautomerase (DCT), II_GBM_14_1 from the receptor tyrosine-protein kinase erbB-2 (ERBB2), and II_GBM_24_1 from the receptor-type tyrosine-protein phosphatase zeta 1 (PTPRZ1). CD4⁺ TILs responded strongly to the peptide pool and proliferated, while the proliferation of CD8⁺ TILs was substantially lower. Proliferating (CFSE^{low}) CD4⁺ T cells were isolated by single cell sorting and expanded to generate CD4⁺ TCCs **(****Fig. 6A****).** Specificity testing of the TCCs showed that 61 CD4⁺ TCCs recognized !!_GBM_12_1, 48 CD4⁺ TCCs recognized II_GBM_14_1, and 31 CD4⁺ TCCs recognized II_GBM_24_1 **(****Fig. 6B****).** Most of the D-neopeptide-specific CD4⁺ TCCs cross-reacted with the N-peptides. This cross-reactivity was seen with all three D-neopeptides **(****Fig. 6C****).** Although most D-neopeptide-specific CD4⁺ TCCs responded slightly less against the N-peptides (lower avidity groups), some CD4⁺ TCCs showed similar (similar avidity groups) or even higher (higher avidity groups) responses to the N-peptides based on proliferation **(****Fig. 6C****),** IFN-γ secretion **(****Fig. 6D****),** and dose titration of the peptides **(****Fig. 6E****).** Importantly, after stimulation with N-peptides, CD4⁺ TCCs showed cytotoxic activity against the autologous tumour cells comparable to those after stimulation with D-neopeptides **(****Fig. 6F****).** Thus, vaccination with D-neopeptides can lead to strong cross-reactive, intratumoural CD4⁺ T cell responses that are directed against the N-peptides. Further, these cross-reactive responses show the desired pro-inflammatory phenotype and induction of tumour cell lysis **(****Fig. 6G****).**

### Summary

Introducing designed AA mutations into tumour-associated peptides as outlined above was remarkably effective with respect to inducing strong intratumoural CD4⁺ T cell responses to 100% of the v-class II-peptides. In the periphery, a gradual increase of memory T cell responses followed successive vaccination rounds. T cells with specificity for v-N-peptides and v-D-neopeptides homed into the tumour, which is supported by the IMC data and the very strong activation responses of TILs. The induced T cells preferentially expressed a proinflammatory Th1 or polyfunctional Th1/2 phenotype and showed cytotoxic activity similar to myelin-specific CD4⁺ T cells in MS patients and virus-specific CD4⁺ T cells in HIV-1 seropositive individuals, functions which are all desired for tumour-specific T cells.

The hypothesis that the introduction of designed AA modifications into unmutated tumour-associated antigens may increase immunogenicity against tumour self-peptides, particularly in a tumour with low mutational load was successful. Putative TCR contact positions were replaced by AA with either conservative- (similar), neutral or non-conservative (different in charge, size, and side chains) physicochemical characteristics. Of note, some D-neopeptides stimulated bulk TILs efficiently and much better than the N-peptides. Both conservative and non-conservative amino acid replacements had stimulatory capacity. Remarkably, even at the level of bulk TILs, activation with D-neopeptides also led to efficient lysis of autologous tumour cells. Experiments using T cell clones showed that individual responding TCRs recognized both the N-peptides and D-neopeptides in three different reactivity patterns, lower-, equal- or even better activation by the N-peptides versus the D-neopeptides. the functional responses included not only activation of proliferation, but also the release of proinflammatory cytokines and cytolysis of autologous tumour cells upon activation with both D-neopeptide and N-peptides. Thus, vaccination with designed neopeptides increases the number of tumour targets in cold tumours and elicits robust intratumoural CD4+ T cell responses not only against the designed neopeptides, but also the unmutated targets expressed by the tumour.

### Materials and Methods

### Ethical considerations and patient information

The goal of the described study was primarily what is referred to as a healing attempt of the patient and to increase the possibility for improving the clinical outcome. A new treatment method was used because the patient was aware of the poor prognosis of standard therapy and wanted to try the vaccination. By definition, this is an individual medical treatment ("compassionate use"), which is not subject to special regulation for medical research according to the Therapeutic Products Act (TPA) or Swiss Federal Human Research Act (HRA). The patient was fully informed about and consented to the steps of the personalized vaccination throughout the treatment period. A 55-year-old man reported headache and memory impairment. A cerebral mass was diagnosed by magnetic resonance imaging (MRI), and the diagnosis of an IDH wild-type glioblastoma was made following surgical resection (1^{st} surgical resection). The patient then received standard of care with temozolomide-based radiochemotherapy and maintenance therapy with temozolomide. Because of further tumour progression, treatment with pembrolizumab, bevacizumab, and an experimental, personalized peptide cocktail vaccination was initiated. Due to further tumour recurrence, the patient underwent a second surgical resection (2^{nd} surgical resection) 14 months after diagnosis. The patient died 4 months later.

### Glioblastoma tissue, leukapheresis, and PBMCs samples

Part of the primary glioblastoma tissue from the 1^{st} surgical resection was immediately frozen at -80°C, which was used for RNA sequencing (RNAseq) and whole exome sequencing (WES). The recurrent glioblastoma tissue obtained during the 2^{nd} surgical resection was partly used for cryopreservation, which was subjected to IMC, and partly for tumour-infiltrating lymphocyte (TILs) isolation. Leukapheresis from the patient was collected prior to any treatment with the main aim to have sufficient numbers of antigen-presenting cells. PBMCs were collected before and after peptide vaccination, which were used for WES, HLA genotyping, or vaccine peptide testing.

### DNA extraction, HLA genotyping, and WES

DNAs were extracted from PBMCs and primary glioblastoma tissue using DNeasy Blood & Tissue Kits (Qiagen, Hilden, Germany). The genotypes of HLA class I (A and B) and HLA class II (DRB1, DRB3, DRB4, DRB5, DPA1, DPB1, DQA1, and DQB1) alleles were determined by high-resolution HLA sequence-based typing (Histogenetics, New York, USA). The WES of PBMCs and the primary tumour was performed at Genomics Facility Basel (Illumina HiSeq 2500, 2×101bp, Capture kit: Agilent SureSelect XT v6+COSMIC). WES reads for tumour and PBMCs were aligned to the human reference genome (hg19) using bwa (arXiv:1303.3997). Secondary alignments as well as duplicate reads were removed using samtools and Picard (http://broadinstitute.github.io/picard/), respectively. Subsequently, we performed Base Quality Score Recalibration and Indel realignment using GATK 3.5. The average coverage for the primary tumour analysis was 255x and 85x for tumour and normal, respectively. On the resulting WES alignments, Strelka, Mutect, and Varscan 2 were used to call somatic variants. Only variants called by at least two of the callers were considered.

### RNA extraction and RNAseq

RNA was extracted from primary glioblastoma tissue using RNeasy Mini Kit (Qiagen, Hilden, Germany). RNA sequencing was performed using HiSeq 4000 System (Illumina, California, USA), 2x150bp, at the Functional Genomics Centre Zurich (FGCZ) and yielded 154,153,378 read pairs. STAR (version 2.4.2) was used to map (2-pass) the RNAseq reads against the human reference genome (hg19). Based on the TCGA-RNAseqv2 pipeline obtained from the public Lineberger Bioinformatics Core database and the STAR alignment quantile normalized gene counts were determined. These quantile normalized gene counts were then used to compare the gene expression in the primary tumour to the TCGA glioblastoma cohort. Gene expression data for the TCGA glioblastoma cohort was downloaded from the Broad GDAC Firehose (Broad Institute TCGA Genome Data Analysis Centre (2016): Analysis Overview for Glioblastoma (Primary solid tumour cohort) - 28 January 2016. Broad Institute of MIT and Harvard. doi:10.7908/C16T0M0N). Gene expression up to 1.5 times the interquartile range greater than the 75th percentile in the cohort gene expression distribution is considered high expression. Gene expression greater than that is considered overexpression.

### Identification of potential TSAs and TAAs

To determine TSAs containing somatic mutations, genomic DNAs were extracted from the primary glioblastoma tissue and PBMCs and analysed using WES. A total of 40 somatic mutants were found and used as TSAs for vaccine peptide prediction. To identify TAAs with high transcription levels in the tumour, RNA was extracted from the primary glioblastoma tissue and analysed using RNA-seq. Over-/highly expressed genes in the primary glioblastoma, when comparing with glioblastoma cohort in TCGA Database, were selected and used as TAAs for vaccine peptide prediction. In addition, a total of 12 glioblastoma-associated TAAs that had been reported previously by immunopeptidome or sequencing analyses were also used for vaccine peptide prediction **(Table 1).** Manual curation of the TSAΓΓAA based on transcription and prior knowledge was performed to exclude peptides with putative high risk of causing damage to vital organs.

### Prediction of TSA/TAA-derived natural peptides

Given the HLA genotype of the patient TSA/TAA-derived peptides were predicted using NetMHCpan 4.0 and the MHC-I and MHC-II binding prediction tools (IEDB_recommended mode) of the IEDB Analysis Resource. According to the binding motif and predicted binding affinity of the peptides for HLA class I and -II molecules, 78 natural peptides (N-peptides) from TSAs or TAAs were selected, which included peptides with a length of 9 AAs for HLA class I molecules (N-class I-peptides, I_MUT/RNA/GBM_X_0) and peptides with a length of 11-13 AAs for HLA class II molecules (N-class II-peptides, II_MUT/RNA/GBM_X_0) as shown in **Table 2.**

### Artificially designed mutation of predicted TSA/TAA-derived natural peptides

To potentially strengthen the stimulatory effect, single AA substitutions were introduced at the potential peptide-TCR contact positions of the N-class I/II-peptides, which were deduced based on SYFPEITHI database, as shown in **Fig. 1****.** The resulting peptides are referred to as designed neopeptides (D-neopeptides) as shown in **Table 2.**

### Design of peptide cocktails for vaccination

Before the 1^{st} peptide cocktail vaccination, the 78 N-peptides and their corresponding D-neopeptides were tested with untouched PMBCs to examine their potential stimulatory effects to T cells. Unfortunately, the PBMCs of the patient responded low or had no response to all peptides, so we chose ~25 peptides from somatic mutants and reported glioblastoma antigens for the 1^{st} and 2^{nd} vaccination. After the 2^{nd} vaccination, the PBMCs of the patient were collected and tested with the vaccine peptides (v-peptides) to examine the effect of the peptide immunotherapy. Meanwhile, the peptides that did not elicit a T cell response after several rounds of vaccination were taken out of the peptide cocktail and replaced by other peptides from the overall set of peptides **(Table 2)** in the subsequent vaccination.

### Histological analyses

Primary and recurrent glioblastomas were formalin-fixed and paraffin-embedded at the Institute of Pathology and Molecular Pathology, University Hospital Zurich. Paraffin-embedded tissue sections were used for H&E (haematoxylin and eosin) staining, immunohistochemical staining for CD3 (Clone: LN10; Dilution: 1:500; Leica Biosystems, Wetzlar, Germany), and imaging mass cytometry (IMC) analyses. For IMC, an antibody panel was designed and used to target markers specific for GBM, immune cell types/subsets, epithelial cells, activation, and proliferation. The preparation and staining of paraffin-embedded tissue sections, acquirement of images, and image data processing were performed as described before.

### Isolation and expansion of TILs

For the isolation of TILs, the surgically resected recurrent glioblastoma was washed with IMDM medium (GE healthcare, Illinois, USA) to remove the blood clots and collagen in a 100mm Petri dish (Sigma-Aldrich, Missouri, USA) and then mechanically cut into small pieces in 5ml digestion solution: IMDM medium (GE healthcare) with 1 mg/ml collagenase A (Roche, Basel, Switzerland) and 50 U DNase (Roche). Small pieces of tissue were digested in 37°C water bath for 1 h with pipetting up and down every 10 min. The digested product was washed twice with IMDM medium (GE healthcare) and passed through a 70µm Cell Strainer (Corning, New York, USA) to get single-cell suspension. The single-cell suspension was centrifuged at 1200rpm for 10 min at 20°C, removed the supernatant, and resuspended cells with 6ml 30% Percoll solution (GE healthcare). TILs were isolated using 30%/78% Percoll gradient centrifugation. For the expansion of TILs, isolated TILs were seeded at 1.5 × 10³ cells/well in 96-well U-bottom plates (Greiner Bio-One, Kremsmunster, Austria) together with 1.5 × 10⁵ irradiated autologous PBMCs (45Gy), 1 µg/ml PHA (REMEL, Thermo Fisher Scientific, Massachusetts, USA), and 20 U/ml human IL-2 (hIL-2 containing supernatant produced by T6 cell line kindly provided by F. Sallusto, IRB, Bellinzona, Switzerland) in RPMI-1640 medium (Sigma-Aldrich, Missouri, USA) containing 5% human serum (Blood Bank Basel, Switzerland), 2 mM glutamine (Thermo Fisher Scientific), 1% (vol/vol) nonessential amino acids (Gibco, Thermo Fisher Scientific), 1% (vol/vol) sodium pyruvate (Gibco), 5µM β-Mercaptoethanol (Gibco), 100 U/ml penicillin (Corning), and 100 µg/ml streptomycin (Corning). The medium was changed by aspirating half of the old medium and adding the same amount of fresh medium containing hIL-2 every 3-4 days for 14 days until cells were fully rested.

### Proliferation assay

All peptides used in this study for stimulation purposes were synthesized with C-terminal amide (Peptides & Elephants, Hennigsdorf, Germany). For peptide stimulation assay of PBMCs, untouched- or CD45RA⁻ PBMCs were tested in the indicated conditions. CD45RA⁻ PBMCs were isolated by negative selection using CD45RA microbeads, human (Miltenyi, Bergisch Gladbach, Germany). In the tests, PBMCs were seeded at 2 × 10⁵ cells/well in 200 µl X-VIVO 15^{™} medium (Lonza, Basel, Switzerland) in 96-well U-bottom plates (Greiner Bio-One), and peptides were then added at a final concentration of 5 µM. Proliferations were measured at day 7 by ³H-thymidine (Hartmann Analytic, Braunschweig, Germany) incorporation assay. Proliferation strength is displayed as counts per minute (cpm) or stimulation index (SI). SI indicates the ratio of cpm in the presence of the peptide versus cpm of the no peptide control. For peptide stimulation assay of TILs, TILs were seeded at 5 × 10⁴ cells/well with 2 × 10⁵ irradiated autologous PBMCs (45Gy) as APCs in 200 µl X-VIVO 15^{™} medium (Lonza) in 96-well U-bottom plates (Greiner Bio-One) and stimulated with peptides at a final concentration of 5 or 10 µM. The anti-human HLA-DR, DP, DQ antibody (TÜ39, kindly provided by Stefan Stevanovic, Tübingen, Germany) was added under some conditions at a final concentration of 10 µg/ml to block the function of HLA class II molecules. Proliferation was measured at day 5 by ³H-thymidine (Hartmann Analytic) incorporation assay. For peptide stimulation assay of TCCs, TCCs were seeded at 2 × 10⁴ cells/well with 1 × 10⁵ irradiated autologous PBMCs (45Gy) in 200 µl X-VIVO 15^{™} medium (Lonza) in 96-well U-bottom plates (Greiner Bio-One) and stimulated with peptides at a final concentration of 5 µM.

### Cytotoxicity assay

Effector TILs were stimulated with pooled peptides for 5 days using irradiated autologous PBMCs as APCs, or effector TCCs were stimulated with peptides for 4 days using irradiated autologous PBMCs as APCs. After stimulation, TILs and TCCs were harvested, washed, and resuspended with X-VIVO 15^{™} medium (Lonza). Target tumour cells were detached with accutase (Thermo Fisher Scientific), washed, and labelled with a PKH26 Red Fluorescent Cell Linker Kit (Sigma-Aldrich) for 3 minutes. After labelling, target tumour cells were washed 3 times with serum containing medium, resuspended with X-VIVO 15^{™} medium (Lonza), adjusted the cell number to 2 × 10⁵ cells/ml, and add 100 µl/well in 96-well U-bottom plates (Greiner Bio-One). Effector TILs or TCCs were added to respective wells containing PKH26-labeled target tumour cells at 1:1, 2:1, 5:1 and 10:1 effector to target (E/T) ratios. Plates were centrifuged at 80 g for 1 min to improve effector-target cell contact and incubated for 24 h. After incubation, plates were centrifuged at 250 g for 5 min, supernatants were removed, and cells were detached with accutase (Thermo Fisher Scientific). Cells were harvested, stained with a LIVE/DEAD^{™} Fixable Near-IR Dead Cell Stain Kit (Thermo Scientific), and analysed using a BD LSR Fortessa to analyse PKH26⁺Near-IR⁺ dead target tumour cells.

### Flow cytometric analysis

For cell surface marker staining, cells were incubated with human IgG (Sigma-Aldrich) and Live/Dead^{®} Aqua (Invitrogen) at 4°C for 30 min and then stained for surface markers using fluorochrome-conjugated antibodies, including FITC anti-human CD69 (FN50), PerCP/Cyanine5.5 anti-human CD3 (HIT3a), APC anti-human CD25 (BC96), APC/Cyanine7 anti-human CD4 (A161A1), Pacific Blue anti-human CD8 (SK1), Alexa Fluor 700 anti-human CD3 (HIT3a), Brilliant Violet 510 anti-human CD8 (SK1), APC anti-human CD194 (CCR4) (L291H4), Brilliant Violet 785 anti-human CD196 (CCR6) (G034E3), Brilliant Violet 711 anti-human CD45RA (HI100), PE anti-human CD294 (CRTH2) (BM16), Brilliant Violet 421 anti-human CD197 (CCR7) (G043H7) antibodies (Biolegend, California, USA), and CD4 monoclonal antibody (S3.5), PE-Texas Red (Thermo Fisher Scientific), at 4°C for 30 min. For intracellular marker staining, after cell surface marker staining, cells were treated with fixation/permeabilization kits (eBioscience, California, United States) and then stained for intracellular markers using fluorochrome-conjugated antibodies (Biolegend), including FITC anti-human/mouse Granzyme B Antibody (GB11), at 4°C overnight. Cells were then washed twice and resuspended with 1× PBS containing 2mM ethylenediamine tetraacetic acid (EDTA, AppliChem, Darmstadt, Germany) and 2% foetal calf serum (FCS, Eurobio, Les Ulis, France). Measurement was performed using LSR Fortessa Flow Cytometer (BD Biosciences) and data were analysed using FlowJo (Tree Star).

### T cell cloning, specificity and cross-reactivity

To generate CD4⁺ TCCs that recognize vaccine peptides, TILs were stained with carboxyfluorescein succinimidyl ester (CFSE, Sigma-Aldrich) and stimulated with pooled peptides, including II_GBM_14_1, II_GBM_12_1, and II_GBM_24_1. Proliferating (CFSE^{low}) CD4⁺ T cells were sorted at day 9 as single cells in 96-well U-bottom plates using a Sorter (SH800S, Sony, Tokyo, Japan) and expanded using irradiated allogeneic PBMCs (45 Gy), 1 µg/ml PHA (Remel), and hIL-2 in RPMI-1640 medium containing 5% human serum (Blood Bank Basel, Switzerland), 2mM L-glutamine (Thermo Fisher Scientific), 100 U/ml penicillin (Corning), 100 µg/ml streptomycin (Corning), and 50 µg/mL gentamicin (Sigma-Aldrich). The medium was changed by aspirating half of the old medium and adding the same amount of fresh medium containing hIL-2 every 3-4 days for 14 days. To identify the target peptides of CD4⁺ TCCs, TCCs were seeded at 2 × 10⁴ cells/well with 1 × 10⁵ irradiated autologous PBMCs (45Gy) in 200 µl X-VIVO 15^{™} medium (Lonza) in 96-well U-bottom plates (Greiner Bio-One) and stimulated with peptides II_GBM_14_0, II_GBM_14_1, II_GBM_12_0, II_GBM_12_1, II_GBM_24_0 or II_GBM_24_1 at a final concentration of 5 µM. Proliferation were measured at day 3 by ³H-thymidine (Hartmann Analytic) incorporation assay, and supernatants were collected for testing IFN-γ using ELISA.

### Cytokine measurement

Supernatants were harvested from TILs at day 5 after stimulation with anti-CD2/CD3/CD28 antibody-loaded MACSibead particles (Miltenyi) or peptides. Cytokines in the supernatants were measured using LEGENDplex Multi-analyte Flow Assay kit (#740722, Biolegend). Supernatants were harvested from TCCs at day 3 after stimulation with peptides. Cytokines in the supernatants were measured using ELISA MAX^{™} Standard Set Human IFN-γ (Biolegend).

### Statistical analysis

Statistical analyses were performed with GraphPad Prism 8.0. Unpaired t-test were used for statistical analyses. Data are expressed as mean or mean ± SEM, and the data were considered statistically significant when differences achieved values of p < 0.05.

## Claims

1. A method of obtaining a neopeptide sequence, the method comprising:
a. in a tumour antigen identification step, providing a tumour antigen polypeptide **characterised in that**:
- an expression level of the tumour antigen polypeptide, or an expression level of the gene encoding the tumour antigen polypeptide exceeds a threshold expression level, wherein the expression level is determined in a tumour sample obtained from a cancer patient; and/or
- a mean expression level of the tumour antigen polypeptide, or a mean expression level of the gene encoding the tumour antigen polypeptide exceeds a threshold expression level, wherein the mean expression level is the mean expression level of the tumour antigen polypeptide, or of the gene encoding the tumour antigen polypeptide determined in a plurality of tumour samples obtained from a cohort of patients diagnosed with a single type of cancer;
b. providing a candidate peptide sequence **characterised in that**:
- it is an 8 to 25 amino acid peptide comprised in a primary amino acid sequence of the tumour antigen polypeptide; and
- it comprises, or consists of, an HLA-binding motif 8 to 11, particularly 9 or 10, more particularly 9 amino acids in length, wherein the HLA-binding motif interacts specifically with a peptide-binding groove of an HLA class I protein, or an HLA class II protein present in a cancer patient;
c. in an HLA-anchor assignment step,
- inputting the candidate peptide sequence into an HLA-anchor prediction algorithm configured to assign a conserved amino acid required for specific association of the HLA-binding motif with the HLA class I protein or the HLA class II protein present in the patient as an HLA-anchor amino acid; and optionally
- assigning as HLA-anchor amino acids:
• an N-terminal amino acid of the HLA-binding motif and any adjacent amino acid, and
• a C-terminal amino acid of the HLA-binding motif and any adjacent amino acid; to provide a candidate peptide comprising, or consisting of HLA-binding motif in which at least 3, particularly 3 to 6 amino acid residues are assigned as HLA-anchor amino acids;
d. in a neopeptide design step, introducing into the candidate peptide sequence one, or two amino acid substitutions to provide a neopeptide sequence,
- wherein at least one of the amino acid substitutions is located within the HLA-binding motif; and
- wherein each of the one, or two amino acid substitutions is located at a position which is not assigned as an HLA-anchor amino acid in the HLA-anchor assignment step.

2. A neopeptide **characterised by** a neopeptide sequence, wherein the neopeptide sequence is **characterised in that**:
- it is a variant of a candidate peptide sequence comprised in a primary amino acid sequence of a tumour antigen polypeptide,
wherein the tumour antigen polypeptide is **characterised in that**:
• an expression level of the tumour antigen polypeptide, or an expression level of the gene encoding the tumour antigen polypeptide exceeds a threshold expression level, wherein the expression level is determined in a tumour sample obtained from a cancer patient; and/or
• a mean expression level of the tumour antigen polypeptide, or a mean expression level of the gene encoding the tumour antigen polypeptide exceeds a threshold expression level, wherein the mean expression level is the mean expression level of the tumour antigen polypeptide, or of the gene encoding the tumour antigen polypeptide determined in a plurality of tumour samples obtained from a cohort of patients diagnosed with a single type of cancer; and
- it comprises, or consists of, an HLA-binding motif 8 to 11, particularly 9 or 10, more particularly 9 amino acids in length, wherein the HLA-binding motif interacts specifically with a peptide-binding groove of an HLA class I protein, or an HLA class II protein present in a cancer patient; and
- it is identical to the candidate neopeptide sequence apart from at least one, particularly apart from only one, or only two amino acid substitutions; and
- wherein at least one of the amino acid substitutions is located within the HLA-binding motif, particularly wherein,
for a neopeptide comprising an HLA-binding motif **characterised by** an even number of amino acids, at least one of the amino acids substitutions is one of the 2, 4 or 6, particularly only of the 2, or 4 central amino acids of the HLA-binding motif, or
for a neopeptide comprising an HLA-binding motif **characterised by** an odd number of amino acids, at least one of the amino acids substitutions is one of the 3, or 5, particularly one of the 3, central amino acids of the HLA-binding motif; and
- wherein when the candidate peptide sequence is inputted into an HLA-anchor prediction algorithm configured to assign an amino acid required for specific association of the HLA-binding motif to the HLA class I protein or the HLA class II protein as an HLA-anchor amino acid, each of the one or more amino acids in the candidate peptide sequence corresponding to the one or more substituted amino acids in the neopeptide sequence, is not assigned as an HLA-anchor amino acid.

3. The method according to claim 1, or the neopeptide according to claim 2, wherein the HLA-binding motif of the neopeptide sequence is **characterised by** a single amino acid substitution in comparison to the HLA-binding motif of the candidate peptide sequence;
particularly wherein the single amino acid substitution is adjacent to an amino acid assigned as an HLA-anchor amino acid.

4. The method according to claims 1 or 3, or the neopeptide according to claims 2 or 3, wherein the neopeptide sequence is **characterised in that**:
- it is 8 to 11, particularly 9 or 10 amino acids in length, and comprises an HLA-binding motif which interacts specifically with a peptide-binding groove of an HLA class I protein; or
- it is 8 to 25, particularly 10 to 15 amino acids in length, and comprises an HLA-binding motif which interacts specifically with a peptide-binding groove of an HLA class II protein.

5. The method according to any one of the claims 1, 3, or 4, or the neopeptide according to any one of the claims 2 to 4, wherein the amino acid substitution is a conservative amino acid substitution selected from the following list:
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable;
- methionine (M) and Q are interchangeable;
- cysteine (C), A and S are interchangeable;
- proline (P), G and A are interchangeable;
- arginine (R) and lysine (K) are interchangeable.
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable; N and D are interchangeable; E and Q are interchangeable;
- methionine (M) and Q are interchangeable;
- cysteine (C), A and S are interchangeable;
- G and A are interchangeable.

6. The method according to any one of the claims 1, 3, or 4, or the neopeptide according to any one of the claims 2 to 4, wherein the amino acid substitution is a non-conservative amino acid substitution not specified in claim 5.

7. A method according to any one of the claims 1, or 3 to 6, or the neopeptide according to any one of the claims 2 to 6, wherein the tumour antigen polypeptide, or the gene encoding the tumour antigen polypeptide is **characterised by**:
• an expression level in a tumour sample obtained from the patient at least 1.5 fold greater than a mean expression level of a plurality of tumour samples obtained from a cohort of cancer patients, wherein each patient in the cohort of cancer patients has been diagnosed with the same type of cancer as the cancer patient; and/or
• a mean expression level in a plurality of tumour samples obtained from a cohort of cancer patients is at least 1.5-fold greater than the mean expression level of a plurality of matched healthy tissue samples obtained from healthy individuals;
particularly wherein the expression level is an mRNA expression level of the gene encoding the tumour antigen polypeptide.

8. A method according to any one of the claims 1, or 3 to 7, or the neopeptide according to any one of the claims 2 to 7, wherein the candidate peptide sequence:
- comprises a tumour-specific somatic mutation, and/or
- is comprised in the primary amino acid sequence of a cancer-associated testis antigen.

9. A method according to any one of the claims 1, or 3 to 8, or the neopeptide according any one of the claims 2 to 8, wherein the cancer is **characterised by** an immunologically cold solid tumour and/or a tumour with a low mutation burden, particularly wherein the cancer is selected from pancreatic cancer, prostate cancer, neuroblastoma, or glioma, more particularly wherein the cancer is glioma.

10. The method according to any one of the claims 1, or 3 to 9, wherein the method further comprises synthesising a neopeptide **characterised by** the neopeptide sequence,
and wherein contacting a cell preparation comprising a population of CD4+ T cell and/or CD8+ T cells isolated from a tumour sample obtained from the cancer patient with the neopeptide **characterised by** the neopeptide sequence evokes:
- an increase in cytokine production by the population CD4+ T cell and/or CD8+ T cells above a threshold level, and/or
- an increase in proliferation of the population CD4+ T cell and/or CD8+ T cells above a threshold level.

11. A vaccine composition comprising at least one neopeptide, wherein the neopeptide is
- **characterised by** a neopeptide sequence obtained by a method as specified in any one of the claims 1, or 3 to 10, and/or
- a neopeptide as specified in any one of the claims 2 to 9,
wherein the neopeptide associates specifically with at least one HLA protein expressed by a cancer patient, particularly wherein the vaccine composition comprises at least 4, and more particularly 6 neopeptides, each associating specifically with a different HLA protein expressed by the cancer patient.

12. A vaccine composition according to claim 11, comprising:
- at least two neopeptides **characterised by** a sequence obtained by a method as specified in any one of the claims 1, or 3 to 10, or
at least two neopeptide according to any one of the claims 2 to 9;
wherein:
- about 50% of the neopeptides interact specifically with an HLA class I protein present in the patient; and
- about 50% of the neopeptides interact specifically with an HLA class II protein expressed by the patient;
particularly wherein the vaccine composition comprises 10-30 neopeptides **characterised by** a sequence obtained by a method as specified in any one of the claims 1, or 3 to 10, or comprises at least two neopeptides according to any one of the claims 2 to 9.

13. A nucleic acid expression vector encoding:
- a peptide **characterised by** the neopeptide sequence obtained by a method as specified in any one of the claims 1, or 3 to 10, and/or
- the neopeptide as specified in any one of the claims 2 to 9.

14. A pharmaceutical composition for use in treating cancer comprising
- a peptide **characterised by** the neopeptide sequence obtained by a method as specified in any one of the claims 1, or 3 to 10, or
- the neopeptide as specified in any one of the claims 2 to 9, or
- the nucleic acid expression vector according to claim 13.

15. The pharmaceutical composition according to claim 14, wherein the cancer is an immunologically cold tumour, particularly wherein the cancer is selected from a list consisting of pancreatic cancer, prostate cancer, neuroblastoma, or glioma, more particularly wherein the cancer is glioma.
